Europäisches Patentamt

European Patent Office

Office européen des brevets

(11) Publication number: **0 627 486 A1**

# EUROPEAN PATENT APPLICATION

(21) Application number: **93401438.2**

(22) Date of filing: **04.06.93**

(51) Int. Cl.5: **C12N 9/02**, C12N 15/53, C12P 21/08, A61K 37/48

(43) Date of publication of application:
**07.12.94 Bulletin 94/49**

(84) Designated Contracting States:
**AT BE CH DE DK ES FR GB GR IE IT LI LU MC NL PT SE**

(71) Applicant: **N.V. INNOGENETICS S.A.**
**Industriepark Zwijnaarde 7,**
**Box 4**
**B-9052 Gent (BE)**

(72) Inventor: **Slegers, Herman**
**Elzendreef 17**
**B-2243 Zandhoven (BE)**
Inventor: **Willems, Jean**
**Wannegemdorp 2**
**B-9770 Kruishoutem (BE)**
Inventor: **Zwijsen, An**
**Heuvelstraat 39**
**B-2530 Boechout (BE)**
Inventor: **Bettadapura, Jayaram**
**Charles de Kerckhovelaan 283**
**B-9000 Gent (BE)**

(74) Representative: **Grosset-Fournier, Chantal Catherine**
**Grosset-Fournier & Demachy s.a.r.l.**
**103 rue La Fayette**
**F-75010 Paris (FR)**

(54) **New extracellular superoxide dismutase, a process for preparing the same and compositions containing the same.**

(57) The invention relates to an extracellular superoxide dismutase B form (EC-SOD B) polypeptide, in substantially pure form, characterized by an amino acid sequence having:
*   at least 50% homology, preferably at least 55% homology, more preferably having at least 60% homology to the amino acid sequence spanning positions 1 to 98 as shown in SEQ ID NO 1 or 2, and/or,
*   at least 90% homology, preferably at least 92% homology, more preferably having at least 95% homology to the amino acid sequence spanning positions 99 to 198 as shown in SEQ ID NO 1 or 2, and/or,
*   at least 52% homology, preferably at least 55% homology, more preferably having at least 60% homology to the amino acid sequence spanning positions 199 to 224 as shown in SEQ ID NO 1 or 2.
The superoxide dismutase of the invention can be used as active substances of pharmaceutical compositions.

EP 0 627 486 A1

The present invention relates to a new extracellular superoxide dismutase, a process for preparing the same, and compositions containing the same.

The technical problem underlying the present invention is to provide a new extracellular superoxide dismutase which shows the ability to dismutate reactive oxygen intermediates produced by activated human neutrophils.

Superoxide dismutase (SOD, EC 1.15.1.1), which represents one of the main defense enzymes against toxic oxygen species, was originally described by McCord & Fridovich (1969) and occurs in several forms. Inside the eukaryotic cell a $Cu^{2+}$-$Zn^{2+}$ enzyme is found in the cytosol and a $Mn^{2+}$-enzyme is located in mitochondria (for a review see Bannister et al., 1987). Both enzymes are conserved throughout evolution. More recently Marklund and coworkers have shown that also several extracellular forms of SOD (referred to as EC-SOD) exist in plasma of human and other mammalian species (Marklund et al., 1982; Hjalmarsson et al., 1987; Tibell et al., 1987). These extracellular forms can be divided into three classes namely A, B and C according to their increasing affinity for heparin (Marklund, 1984; Karlsson & Marklund, 1987; Karlsson & Marklund, 1988).

Until now only the human extracellular SOD C (EC-SOD C) was completely purified, sequenced, cloned and expressed. Many patent applications and specifications have delt with these applications (f.i. EP-A-0 492 447, EP-B-0 236 385).

This EC-SOD C avidly binds to anchorage-dependent cell lines but has almost no affinity for blood cells, including neutrophils Karlsson & Marklund, 1989). In the body, the major portion of EC-SOD C binds to heparin sulfate proteoglycan in the glycocalyx of cell surfaces and in the connective tissue forming an equilibrium with the extracellular fluid phase. EC-SOD C is known to bind to endothelial cell surfaces and most likely also to surfaces of other cell types, whereas EC-SOD of the subtypes A and B have been proposed to exist mainly in the extracellular fluid phase (Adachi & Marklund, 1989; Karlsson & Marklund, 1988).

According to Hjalmarsson et al. (1987) the binding of EC-SOD C to sulfated proteoglycan and heparin is due to a hydrophilic C-terminal sequence containing 6 arginine and 3 lysine residues in the last 21 amino acids. The C form is absent in rat and therefore, in this species the B form is the only EC-SOD that is able to bind to heparin (Karlsson & Marklund, 1988). It has been suggested that A and B forms are derived from the C form by post-translational processing at the carboxyl terminus (Adachi et al., 1991; Sandstrom et al., 1992).

According to Karlsson & Marklund (1988), plasma EC-SOD from man, pig, rat, rabbit, guinea pig and mouse with apparent molecular masses around 165 kDa as determined by Gel chromatography are probably tetrameric. Rat plasma EC-SOD appeared to have a molecular mass of about 97 KDa and might be dimeric.

Furthermore, EC-SOD of any type is known to be secreted by fibroblasts and glial cells but not by endothelial or epithelial cells. Glioma cells do not always secrete SOD (Marklund, 1990).

The aim of the present invention is to provide purified extracellular superoxide dismutase (EC-SOD) B form polypeptides, which dismutate reactive oxygen intermediates produced by activated neutrophils, and which interact with neutrophils and possibly also with other types of blood cells.

Another aim of the present invention is to provide the nucleotide and amino acid sequence of this EC-SOD B form.

Another aim of the present invention is to provide pharmaceutical compositions which can be used, for instance, in the treatment of diseases or disorders connected with the presence or the formation of superoxide radicals.

All of these aims are achieved by the polypeptides of the invention.

The present invention relates in particular to an extracellular superoxide dismutase B form (EC-SOD B) polypeptide, wherein said EC-SOD B polypeptide is characterized as any amino acid sequence having:

* at least 50% homology, preferably at least 55% homology, more preferably having at least 60% homology to the amino acid sequence spanning positions 1 to 98 as shown in SEQ ID NO 1 or 2, and/or,
* at least 90% homology, preferably at least 92% homology, more preferably having at least 95% homology to the amino acid sequence spanning positions 99 to 198 as shown in SEQ ID NO 1 or 2, and/or,
* at least 52% homology, preferably at least 55% homology, more preferably having at least 60% homology to the amino acid sequence spanning positions 199 to 224 as shown in SEQ ID NO 1 or 2.

The expression "substantially pure form" refers to a purity grade of at least 90%, preferably 95%, and more particularly of 98%, as determined by 2D-PAGE.

The Examples section of the present invention teaches a protein isolated from conditioned medium of rat glioma cells. Partial amino acid sequence determination showed that the polypeptide of the invention resembles the human extracellular superoxide dismutase C (Hjalmarsson et al., 1987). Analysis of formic acid, CNBr, trypsin and hydroxylamine-generated peptides revealed several sequence differences especially in the regions outside the active center of the enzyme in comparison with the human extracellular superoxide dismutase C and intracellular superoxide dismutases. However, the regions flanking the active center domain have substantially less homology.

The determined amino acid sequences were used to generate an EC-SOD specific cDNA probe from C6 mRNA by PCR and to isolate an EC-SOD cDNA clone from a lambda gt11 cDNA library. The full size cDNA sequence confirmed the determined amino acid sequences. The isolated rat EC-SOD is more than 90% homologous to human EC-SOD C in its active center, has an almost identical hydrophilic sequence in the heparin-binding site located in the 15 C-terminal amino acids, but is substantially less homologous in the N-terminal sequence and the C-terminal region flanking the active center.

Furthermore, the protein of the invention elutes at a lower salt concentration (300-400 mM) from heparin-Sepharose compared to human EC-SOD C (500 mM). It has a considerable difference in pI (its pI varies between 6.1 and 7.3, while human EC-SOD C is acid stable and has a pI of about 4.5 (Marklund & Karlsson in "Antioxidants in therapy and prevetive medicine" Ed. I. Emerit, Plenum Press, New York, 1990, p. 1-4) and, contrary to the human C form, it is acid labile and has a binding affinity for human neutrophils. Since, Karlsson & Marklund (1988) have demonstrated that in rat only EC-SOD A and B forms exist and the EC-SOD of the present invention elutes at a NaCl concentration (300-400 mM) from heparin-Sepaharose comparable to the previously suggested rat EC-SOD B (Karlsson & Marklund, 1988), the protein of the present invention was tentatively named the rat EC-SOD B form.

Finally, unlike the extracellular C form which exhibits no binding to neutrophils (Karlsson & Marklund, 1989), the purified B form is able to bind to human neutrophils, even at physiological amounts of neutrophils (i.e. 1 million neutrophils/ml of blood). This implies possible therapeutic effects not shown by other superoxide dismutases.

The present invention relates more particularly to a polypeptide as defined above, wherein said EC-SOD B polypeptide comprises an amino acid sequence having at least 67% homology, preferably at least 70% homology, most preferably 75% homology to the 224 amino acids long mature polypeptide amino acid sequence as shown in SEQ ID NO 1 or 2.

The polypeptides of the invention such as defined above can be either under a monomeric form, or under a polymeric form, particularly under a dimeric form.

The present invention relates even more particularly to a polypeptide as defined above wherein said EC-SOD B polypeptide is further characterized by its biological activity involving:
-  the dismutation of reactive oxygen intermediates produced by activated human neutrophils and
-  its binding affinity to human neutrophils.

The expression "biological activity involving dismutation of reactive oxygen intermediates produced by activated human neutrophils" (also hereafter designated by SOD activity) refers to the fact that like the rat EC-SOD B form polypeptide, the EC-SOD B polypeptide of the invention is able to inhibit the respiratory burst of activated neutrophils measured as inhibition of for instance PMA-induced superoxide production by human neutrophils.

One unit of inhibitory activity is defined as the dilution which gives a 50% reduction of the PMA-induced luminescence value. The 50% reduction is measured from a calibration curve obtained with serial dilutions of a test sample. Using commercial $Cu^{2+}$-$Zn^{2+}$ SOD it was calculated that the assay described in the Examples section is about 6 times more sensitive than the classical cytochrome C reduction test, which implies that one unit in the described assay is equivalent to the activity of approximately 50 ng of intracellular SOD and 5.8 ng of rat EC-SOD (see Table 2, Examples section).

The expression "binding affinity to human neutrophils" refers to the fact that binding of the extracellular SOD of the present invention was observed to human neutrophils as demonstrated in the Examples section. This biological activity may be measured by a luminescence assay as described in the Examples section, or by any other method comprised in the art.

The term "neutrophil" refers to blood cells belonging to the the group of polymorphonuclear leucocytes (PMN). The EC-SOD molecules of the present invention may also have an affinity for other types of blood cells, such as leucocytes and platelets or other cells present in the afflicted areas (e.g. the vascular endothelial cells of the vessel walls, tissue mononuclear phagocytes, and mast cells). The blood leucocytes may be subdivided in two major groups: polymorphonuclear leucocytes (PMN, also referred to as granulocytes) and mononuclear leucocytes. The PMNs are further subclassified into neutrophils, eosinophils, and basophils.

The PMNs are implicated at an early stage in the inflammatory response and predominate at focal points of acute inflammation; they make up some 40-60% of the white blood cells and are relatively short-lived. They migrate through the blood vessel walls (post-capillary venules) into the affected areas.

The terms "neutrophils" and "PMN" are reviewed by Densen and Mandell (1985) and by Becker (1988).

Besides having an activity of dismutating the extracellular production of reactive oxygen intermediates produced by activated neutrophils, the polypeptides of the invention may also have an activity as radical scavengers (e.g. as produced by eosinophils). The possible effects of the polypeptide of the invention on monocytes, macrophages and/or eosinophils may be assayed by any of the techniques known in the art, for instance those reviewed in "Inflammation. Basic priciples and clinical correlates" Eds. Gallin, Goldstein & Snydermann, Raven Press (1988). The possible effects on B-cells may be assayed by any one of the techniques known in the art, such as described by Furakawa et al., 1992

The polypeptides of the invention, and particularly the shorter peptides (from about 10 to about 50 amino acids) amongst them, can be prepared by classical chemical synthesis. The synthesis can be carried out in homogeneous solution or in solid phase.

For instance, the synthesis technique in homogeneous solution which can be used is the one described by Houbenweyl in the book entitled "Methode der organischen chemie" (Method of organic chemistry) edited by E. Wunsh, vol. 15-I et II. THIEME, Stuttgart 1974.

The polypeptides of the invention can also be prepared in solid phase according to the methods described by Atherton and Shepard in their book entitled "Solid phase peptide synthesis" (IRL Press, Oxford, 1989).

The polypeptides according to this invention can also be prepared by means of recombinant DNA techniques as documented below.

The present invention relates even more particularly to a polypeptide as defined above wherein said EC-SOD B polypeptide is further characterized as:

* having a native molecular mass of about 85 kDa ± 20 kDa as determined by gelfiltration on a Toyopearl HW 55S and on a Superose 12 column, and with a subunit molecular weight ranging from about 30 kDa to about 38 kDa as determined by SDS-PAGE,
* having an isoelectric point ranging from about 6.1 to about 7.3, as determined by isolelectrofocusing,
* eluting from a heparin-Sepharose column between 0.1 and 0.5 M NaCl, and preferably between 0.3 and 0.4 M NaCl,
* being secreted by mammalian cells,
* having binding affinity for human neutrophils.

The Examples section of the present invention demonstrates the purification and characterization of an EC-SOD B form derived from the culture medium of rat glioma cells which binds to and strongly reduces the level of superoxide radicals released by activated neutrophils. It is, however, to be understood that the same polypeptide may also be isolated from other cell types than glioma cells, such as fibrosarcoma, neuroblastoma or fibroblast cells as well as cells originating from other mammals such as humans.

The polypeptide of the invention was purified to homogeneity as indicated by the presence of a doublet band on one-dimensional SDS-PAGE and a set of spots on two-dimensional electrophoresis which migrated in the pI range of 6.1 to 7.3. The rat EC-SOD B of the invention was identified as having a native molecular mass of about 85 kDa ± 20 kDa (as determined by gelfiltration on a Toyopearl HW 55S and on a Superose 12 column) which is significantly less than the molecular weight reported for human EC-SOD C (Mr of 140,000, Marklund et al., 1982). Rat EC-SOD B of the present invention has a subunit molecular mass of 30 to 38 kDa, as determined by SDS-PAGE. The variation between the subunit molecular mass and the native molecular mass is most probably due to modification (e.g. glycosylation, phosphorylation, sialylation) of the same protein. This difference in molecular weight according to the determination method used infers the existence of a dimer.

The polypeptide of the invention can be either in the form of a monomer defined by the above-defined subunit as determined by SDS-PAGE, or in the form of a polymer of said monomer, and particularly in the form of a dimer.

The binding activity of the polypeptide of the invention to neutrophils may be determined according to a neutrophil binding assay as mentioned in the Examples section or by any other assay comprised in the art.

According to a preferred embodiment, the present invention relates to a polypeptide as defined above further characterized by the 224 amino acid sequence as represented in SEQ ID NO 1 or 2.

According to a preferred embodiment, the present invention relates to a polypeptide as defined above, in a glycosylated form.

According to another embodiment, the present invention relates to a polypeptide as defined above liable to be obtained according to a process including at least the following steps:

- growing cells known to secrete the polypeptide to be isolated and purified in a suitable culture medium and harvesting the cells to recover the culture medium,
- performing a heparin-Sepharose chromatography with the recovered culture medium and collecting the fractions showing the SOD biological activity and/or the neutrophil binding activity and eluting between 0.1 and 0.5 M NaCl, preferably between 0.3 and 0.4 M NaCl,
- performing a hydrophobic interaction chromatography on the biologically active fractions obtained after heparin-Sepharose chromatography and collecting the fractions showing the SOD biological activity and/or the neutrophil binding activity and eluting between 400 nM and 200 nM $(NH_4)_2SO_4$ in 50 mM sodiumphosphate (pH 7.0),
- performing a heparin-HPLC chromatography with the biologically active fractions obtained from the hydrophobic interaction chromatography and collecting the fractions having the SOD activity and/or the neutrophil binding activity between 250 mM and 300 mM NaCl, 50 mM Tris-HCl pH 7.1,
- performing a reversed-phase HPLC chromatography with the biologivally active fractions obtained from the heparin-HPLC chromatography, preferably C4, and isolating the fractions showing the SOD biological activity, and/or the neutrophil binding activity.

According to one possible embodiment of the invention, the cells used are a rat glioma C6 cell line (ATCC CCL 107). These rat cells are seeded in serum-free medium supplemented with 30 nM sodium selenate with said cells being allowed to grow to confluency and maintained for 48 to 72 hours in said medium and are harvested from the conditioned medium by centrifugation.

In the reversed-phase HPLC chromatography, the fractions showing the biological activity are advantageously eluted between 30 and 35% acetonitrile.

According to another preferred embodiment the present invention relates to a polypeptide or peptide comprising a contiguous sequence of at least 10 amino acids of an extracellular SOD B form polypeptide as defined above, with said polypeptide or peptide presenting at least one of the biological activities of the extracellular B form as defined above, and with said peptide or polypeptide containing in its contiguous sequence at least one amino acid different from the corresponding region of human EC-SOD C.

According to a preferred embodiment, the present invention relates to a polypeptide as defined above comprising a contiguous sequence derived from the 224 amino acids long mature peptide amino acid sequence as shown in SEQ ID NO 1 or 2, more particularly a polypeptide comprising a contiguous sequence of at least one of the following regions of the 224 amino acids long mature peptide:

- amino acids spanning position 199 to 213,
- amino acids spanning position 1 to 55,
- amino acids spanning positions 53 to 72,
- amino acids spanning positions 71 to 89,
- amino acids spanning positions 87 to 102,
- amino acids spanning positions 100 to 108,
- amino acids spanning positions 119 to 127,
- amino acids spanning positions 133 to 141,
- amino acids spanning positions 138 to 156,
- amino acids spanning positions 157 to 166,
- amino acids spanning positions 173 to 181,
- amino acids spanning positions 181 to 189,
- amino acids spanning positions 195 to 214,
- amino acids spanning positions 219 to 224.

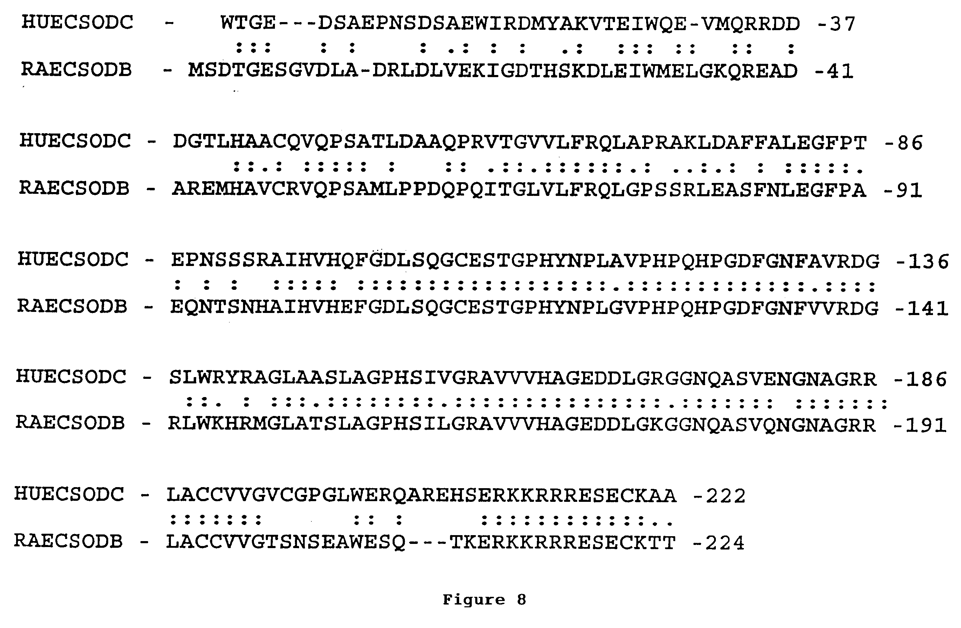

The above given list of regions of the EC-SOD B form polypeptide as represented in SEQ ID NO 1 or 2 is characterized by the fact that they are chosen amongst the regions with fewest homology to the human EC-SOD C amino acid sequence. An amino acid comparison between the rat EC-SOD B form and the human EC-SOD C form is given in Figure 8.

The present invention relates to a polynucleic acid in substantially isolated form comprising a contiguous sequence of at least 10 nucleotides selected from:

(a) the polynucleic acid sequences which code for any of the EC-SOD B polypeptides as defined above or,

(b) the polynucleic acid sequences which hybridize to any of the polynucleic acids as defined in (a), or,

(c) the polynucleic acid sequences which are degenerate as a result of the genetic code to the polynucleic acid sequences as defined in (a) or (b) and which either code for a polypeptide as defined above, or hybridize to a polynucleic acid which codes for a polypeptide as defined above.

The term "hybridizes to" refers to conventional hybridization conditions known to the man skilled in the art, preferably to stringent hybridization conditions.

The polynucleic acids of the invention are to be understood as also comprising the degenerate nucleic acids of the nucleic acids coding for the above-defined polypeptides of the invention.

The term "polynucleic acid" corresponds to either double-stranded or single-stranded cDNA or genomic DNA, or RNA.

The polynucleic acid according to this embodiment of the invention can be determined by means of a process comprising the steps of:

- preparing mRNA from mammalian cells, for instance from rat C6 glioma cells and/or human WI-38 foetal lung fibroblast cells (step 1),
- performing an amplification reaction, such as PCR, of mRNA, with primers essentially consisting of, or comprising a nucleotide sequence containing at least part of a nucleotide sequence encoding an extracellular superoxide dismutase B form as defined above, preferably from the nucleotide sequence of rat EC-SOD B as represented in SEQ ID NO 1, to obtain amplified products (step 2),
- screening of the mammalian cDNA libraries to obtain full length cDNA clones by using the amplified products obtained in the previous step, as a probe for hybridization (step 3),
- screening of a mammalian genomic library to obtain the full length genomic clone by using the above-mentioned amplified products of step 2 or cDNA clones obtained in step 3 as a probe for hybridization (step 4).

The present invention relates to a polynucleic acid as defined above comprising a contiguous sequence of at least 10 nucleotides selected from:

(a) the polynucleic acid sequence as shown in SEQ ID NO 1, or,

b) the polynucleic acid sequences which hybridize to the polynucleic acid as shown in SEQ ID NO 1, or,

(c) the polynucleic acid sequences which are degenerate as a result of the genetic code to the polynucleic acid sequences as shown in SEQ ID NO 1, or to the polynucleic acid sequences which hybridize to the polynucleic acid sequences as shown in SEQ ID NO 1.

According to this embodiment, the present invention relates to any nucleotide sequence which gives rise to the polypeptide sequence as represented in SEQ ID NO 1 or 2.

The present invention relates more particularly to a polynucleic acid sequence as defined above comprising a cDNA or a full length genomic clone encoding any of the polypeptides as defined above, with said clone being obtained by a process comprising essentially the following steps:

- preparing mRNA from mammalian cells (step 1),
- performing an amplification reaction of mRNA with primers essentially consisting of, or comprising at least part of a nucleotide sequence encoding a polypeptide as defined above, to obtain amplified products (step 2),
- screening of a mammalian cDNA library to obtain full length cDNA clones by using the amplified products obtained in the step 2, as a probe for hybridization (step 3),
- screening of a genomic library to obtain the full length genomic clone by using the above-mentioned amplified products of step 2 or cDNA clones obtained in step 3 as a probe for hybridization (step 4).

The present invention relates also to a polynucleic acid sequence comprising at least 10 nucleotides as defined above, for use as a specific hybridization probe for detecting the presence of a polynucleic acid encoding any of the polypeptides as defined above, or the complement thereof.

The term probe refers to single stranded sequence-specific oligonucleotides which have a sequence which is complementary to a target sequence to be detected or cloned. Probes may be labelled according to any of the techniques known in the art. Preferably these probes are about 5 to 50 nucleotides long.

The probes according to this aspect of the invention include probes used for hybridizing to and/or cloning of genes, or parts thereof, encoding extracellular superoxide B form dismutase, provided that they show:

* at least 63%, more preferably at least 65%, and most preferably more than 70% of nucleotide sequence homology with the 1730 bp nucleotide sequence given in SEQ ID NO 1, and/or,
* at least 59%, more preferably at least 63%, and most preferably more than 70% of nucleotide sequence homology with the 5' untranslated region (1-133) of the 1729 bp nucleotide sequence given in SEQ ID NO 1, and/or,
* at least 63%, more preferably at least 65%, and most preferably more than 70% of nucleotide sequence homology with the signal peptide and mature peptide region (nucleotide position 134-437) as shown in SEQ ID NO 1, and/or,
* at least 81%, more preferably at least 85%, and most preferably more than 90% of nucleotide sequence homology with the SOD active center encoding the region spanning positions 138 to 868 of

6

the nucleotide sequence as shown in SEQ ID NO 1, and/or,

* at least 53%, more preferably at least 55%, and most preferably more than 60% of nucleotide sequence homology with the 3' untranslated region (869-1729) of the nucleotide sequence as shown in SEQ ID NO 1,

Probes according to this aspect of the invention may be used to determine the amount of EC-SOD B polypeptide according to the present invention in all types of test samples.

In a most advantageous embodiment, the above-mentioned probes may also be used to isolate the human counterpart of the rat EC-SOD B form as disclosed in the Examples section.

According to the hybridization solution (SSC, SSPE, etc.), these probes should be hybridized at their appropriate temperature in order to attain sufficient specificity.

Possible probes for screening a human WI38 foetal lung fibroblast cell line cDNA library (Clontech), or any other human cell line producing an EC-SOD B form, for obtaining the human counterpart of the rat EC-SOD B form are included in the following list:

Nucleotide position 195 to 348 of SEQ ID NO 1, nucleotide position 360 to 489 of SEQ ID NO 1, nucleotide position 783 to 864 of SEQ ID NO 1, nucleotide position 1 to 133 of SEQ ID NO 1 and nucleotide position 866 to 1729.

The present invention relates more particularly to a polynucleic acid sequence as defined above, for use as a primer for amplification of a polynucleic acid encoding any of the polypeptides as defined above, or the complement thereof.

The term primer refers to a single stranded DNA oligonucleotide sequence capable of acting as point of initiation for synthesis of a primer extension product which is complementary to the nucleic acid strand to be copied. The length and the sequence of the primer must be such that they allow to prime the synthesis of the extension product. Preferably the primer is about 5 to 50 nucleotides. Specific length and sequence will depend on the complexity of the required DNA or RNA targets, as well as on the conditions of primer use such as temperature and ionic strength. The amplification method used can be any method known in the art.

According to another embodiment, the present invention relates to a recombinant vector, particularly for cloning and/or expression, with said recombinant vector comprising a vector sequence, itself comprising a coding sequence which comprises a DNA polynucleotide as defined above, and wherein the coding sequence is operably linked to a control sequence capable of providing for the expression of the coding sequence by the specific host cell.

The term "vector" may comprise a plasmid, a cosmid, a phage, or a virus.

The term "operably linked" refers to a juxtaposition wherein the components are configured so as to perform their usual function. Thus, control sequences operably linked to a coding sequence are capable of effecting the expression of the coding sequence.

The term "control sequence" refers to those sequences which control the transcription and/or translation of the coding sequences; these may include but are not limited to promoter sequences, transcriptional initiation and termination sequences, and translational initiation and termination sequences. In addition, control sequences refer to sequences which control the processing of the polypeptide encoded within the coding sequence; these may include, but are not limited to sequences controling secretion, protease cleavage, and glycosylation of the polypeptide.

It is to be understood that besides containing nucleic acid sequences from rat origin as shown in the Examples section, this vector may also consist of, or contain sequences encoding the human equivalent of the rat EC-SOD B form.

It should also be understood that the signal sequence (and the signal peptide encoded by it) in itself forms an aspect of the invention, and it is contemplated that it may be inserted upstream of DNA sequences coding for other proteins or peptides so as to obtain secretion of the resulting products from the cells.

According to yet another embodiment, the present invention relates to a host cell transformed by a recombinant vector as defined above.

In order to carry out the expression of the polypeptides of the invention in bacteria such as *E. coli* or in eukaryotic cells such as in *S. cerevisiae*, or in cultured vertebrate or invertebrate hosts such as insect cells, Chinese Hamster Ovary (CHO), COS1, BHK, and MDCK cells, the following steps are carried out:

- transformation of an appropriate cellular host with a recombinant vector, in which a nucleotide sequence coding for one of the polypeptides of the invention has been inserted under the control of the appropriate regulatory elements, particularly a promoter recognized by the polymerases of the cellular host and, in the case of a prokaryotic host, an appropriate ribosome binding site (RBS), enabling the expression in said cellular host of said nucleotide sequence,

- culture of said transformed cellular host under conditions enabling the expression of said insert.

According to yet another embodiment, the present invention relates to a recombinant polypeptide produced by transforming a recombinant vector as defined above into a suitable prokaryotic or eukaryotic host as defined above, and culturing said transformed cellular host under conditions enabling the expression of said insert.

According to another embodiment, the present invention relates to a method of producing a recombinant polypeptide as defined above comprising incubating a host cell as defined above under conditions that provide for the expression of the coding sequence.

According to yet another embodiment, the present invention relates to an antibody, particularly a monoclonal antibody, characterized in that it is specifically directed against an antigenic determinant of a polypeptide as defined above.

The monoclonal antibodies of the invention can be produced by any hybridoma liable to be formed according to classical methods from splenic cells of an animal, particularly from a mouse or rat, immunized against the EC-SOD B form polypeptides according to the invention, or fragments thereof as defined above on the one hand, and of cells of a myeloma cell line on the other hand, and to be selected by the ability of the hybridoma to produce the monoclonal antibodies recognizing the polypeptides which has been initially used for the immunization of the animals.

The antibodies involved in the invention can be labelled by an appropriate label of the enzymatic, fluorescent, or radioactive type.

The monoclonal antibodies according to this preferred embodiment of the invention may be humanized versions of mouse monoclonal antibodies made by means of recombinant DNA technology, departing from parts of mouse and/or human genomic DNA sequences coding for H and L chains or from cDNA clones coding for H and L chains.

Also fragments derived from these monoclonal antibodies such as Fab, F(ab)'2 and recombinant ssFv ("single-chain variable fragment"), providing they have retained the original binding properties, form part of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses.

Alternatively the monoclonal antibodies according to this preferred embodiment of the invention may be human monoclonal antibodies. Such human monoclonal antibodies are prepared, for instance, by means of human peripheral blood lymphocytes (PBL) repopulation of severe combined immune deficiency (SCID) mice (for recent review, see Duchosal et al. 1992).

The invention also relates to the use of the proteins of the invention, or peptides derived there from for the selection of recombinant antibodies by the process of repertoire cloning (Perrson et al., 1991).

According to yet another embodiment, the present invention relates to an anti-idiotype antibody raised against the antibody as defined above.

The term "anti-idiotype antibodies" refers to monoclonal antibodies raised against the antigenic determinants of the variable region of monoclonal antibodies themselves raised against the EC-SOD B polypeptides, muteins, or peptide fragments thereof. These antigenic determinants of immunoglobulins are known as idiotypes (sets of idiotopes) and can therefore be considered to be the "fingerprint" of an antibody (for review see de Préval, 1978; Fleishmann and Davie,1984). The methods for production of monoclonal anti-idiotypic antibodies have been described by Gheuens and McFarlin (1982). Monoclonal anti-idiotypic antibodies have the property of forming an immunological complex with the idiotype of the monoclonal antibody against which they were raised. In this respect the monoclonal antibody is often referred to as Ab1, and the anti-idiotypic antibody is referred to as Ab2. These anti-idiotype Ab2s may be used as substitutes for the polypeptides of the invention or as competitors for binding of the polypeptide of the invention to their target.

A humanized version of an anti-idiotypic monoclonal antibody bearing the same catalytic activity as the EC-SOD B form polypeptides of the present invention is particularly important as active substance in a pharmaceutical composition because of its activity in combination with its expected long half life in circulation specific for a human monoclonal antibody.

The invention also relates to antisense peptides derived from the polypeptides of the invention or derived from the nucleic acid sequences of the invention.

More particularly, the term "antisense peptide" is reviewed by Blalock (1990) and by Roubos (1990). In this respect, the molecular recognition theory (Blalock, 1990) states that not only the complementary nucleic acid sequences interact but that, in addition, interacting sites in proteins are composed of complementary amino acid sequences (sense ligand with receptor or sense ligand with antisense peptides). Thus, two peptides derived from complementary nucleic acid sequences in the same reading frame will show a total interchange of their hydrophobic and hydrophilic amino acids when the amino terminus of one is aligned with the carboxy terminus of the other. This inverted hydropathic pattern might allow two such peptides to assume complementary conformations responsible for specific interaction.

The antisense peptides can be prepared as described in Ghiso et al. (1990). By means of this technology it is possible to logically construct a peptide having a physiologically relevant interaction with a known peptide by simple nucleotide sequence analysis for complementarity, and synthesize the peptide complementary to the binding site.

According to another embodiment, the present invention relates to any of the polypeptides as defined above, for providing superoxide dismutase therapy in any illness state related to the production of superoxide radicals, more particularly any illness state where binding to neutrophils is highly advantageous.

SOD activity for potential therapeutic applications has been demonstrated or suggested for a large range of disorders.

First of all, SOD has been shown to protect against tissue-damage in inflammatory diseases.

The other large potential area of application for SOD is as protective factor against tissue damage caused by ischemia followed by reperfusion, such as reperfusion damage in the case of myocardial infarct.

SOD's also show interesting protective effects in connection with other pathologies such as pancreatitis or ulcerative colitis. It has also been suggested that treatment with SOD is effective against burns, or to increase the viability of excised isolated organ transplants, or for treatment of ulcers and renal disease.

SOD's have also been shown to have a protective effect against lung damage caused by hyperoxia, such as infantile respiratory distress syndrome or bronchopulmonary dysplasia. Also SOD has been reported to reduce the frequency of intraventricular brain hemorrhage or cerebrovascular damage following hypotension. Administration of SOD is also believed to be able to relieve vasoconstriction resulting from elevated superoxide radical levels. SOD may also have a protective effect against the tissue damage caused by the release of superoxide radicals during prostaglandin synthesis in cases such as severe increase in blood pressure. Beneficial effects of the use of SOD in cornea transplantations, retinopathy and ophtalmic surgical procedures have also been reported.

SOD is also used to treat artherosclerosis or patients with glutathion deficient neutrophils.

SOD has has also been suggested to give a protective action in the occurence of various types of auto-immune diseases, such as rheumatoid arthritis, systemic lupus erythematosus.

Other activities of SOD include scavenging superoxide anions due to exposure of a host to various superoxide-inducing agents such as radation, paraquat, etc.; treatment of certain degenerative diseases such as emphysema; food preservation and the like.

Up till now, SOD has been successfully used for the treatment of rheumatoid arthritis, Duchenne's muscular dystrophy and radiation induced cystitis. Trials are ongoing for the treatment of myocardial infarction, burns, polytraumatous shock, liver transplants, respiratory dysplasia in prematures, ulcerative colitis, osteoarthritis or bronchopulmonary dysplasia, and many others.

As an extracellular polypeptide, the EC-SOD B of the present invention mainly will have a function to protect plasma components or the outer surface of cells against the toxic effects of superoxide radicals or other oxygen radicals.

Since the EC-SOD B form of the present invention has the advantage over the EC-SOD C form in that it binds to neutrophils, the EC-SOD B form of the present invention may be of particular use in treating such conditions of superoxide radicals production where a binding to neutrophils of the SOD enhances such a treatment.

The present invention also relates to pharmaceutical compositions, containing as active subtance, anyone of the polypeptides as defined above, or of the anti-idiotype antibodies as defined above in association with a pharmaceutically acceptable vehicle.

The EC-SOD B form of the present invention may be used for the same purposes as the other SODs or more particularly for purposes where binding of SOD to neutrophils is required. Compositions containing the SOD of the present invention may be used in human or veterinary medicine to treat (i.e. cure, alleviate or prevent) a number of conditions. The mode of administration of these pharmaceutical compositions is orally or preferably parenteral, i.e., intravenous, intraarterially, intraperitoneal, intramuscular, or subcutaneous, with the intravenous administration being preferred. When used to treat tissues, the active substances of these pharmaceutical compositions may also be administered alone, without a carrier vehicle; however, they may also be administered with pharmaceutically acceptable non-toxic carriers or diluents, the proportions of which are determined by the suitability and chemical nature of the particular carrier.

The dose of SOD-B form of the present invention will depend on the nature of the individual being treated.

The invention also relates to the use of a polypeptide as defined above or of an anti-idiotype antibody as defined above, as active substance for the preparation of a drug useful in the treatment of all types of lung tissue damage, such as hyperoxia, respiratory distress syndrome, emfysema, or the treatment of radiation damage or burns or ulcers, retinopathy in premature infants, ischemic reperfusion damage, acute myocardial infarction, cerebrovascular damage, organ transplants, atherosclerosis, patients carrying glutathion synthase deficient neutrophils, all types of symptoms associated with aging, such as cataract, all types of inflammatory and auto-immune diseases, such as rheumatoid arthritis, or useful in the cold storage of organs.

The invention also relates to a process for purification of a polypeptide liable to be obtained according to the above-defined process, comprising an affinity chromatography using immobilized antisense peptides of the invention, or immobilized antibodies raised against the polypeptides of the invention.

According to this aspect of the invention, an affinity chromatography system can be used wherein either the antisense peptides or the antibodies raised against the polypeptides of the invention are immobilized on a matrix according to any of the methods comprised in the art.

## BRIEF DESCRIPTION OF THE FIGURES

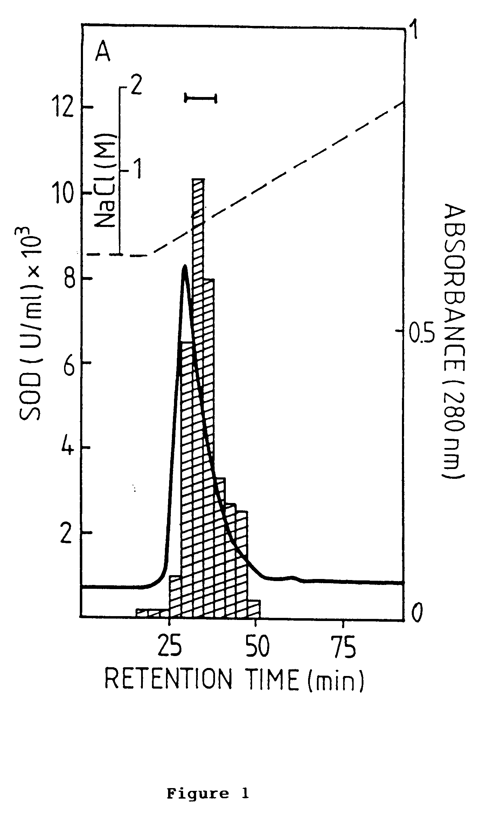

Figure 1: Purification of rat EC-SOD B by means of Heparin-Sephaose 4B affinity chromatography. The eluted fractions from hydroxyapatite were dialysed against 50 mM Tris-HCl (pH 7.5) and divided in six equal parts. Each part was chromatographed separately on a heparin-Sepharose column and the activity was eluted with a NaCl gradient (---). Fractions of 3 ml were collected and assayed for SOD-activity. The activity is represented in a block diagram. OD $_{280nm}$ (_). The fractions under the horizontal bar were pooled and subjected to hydrophobic interaction chromatography.

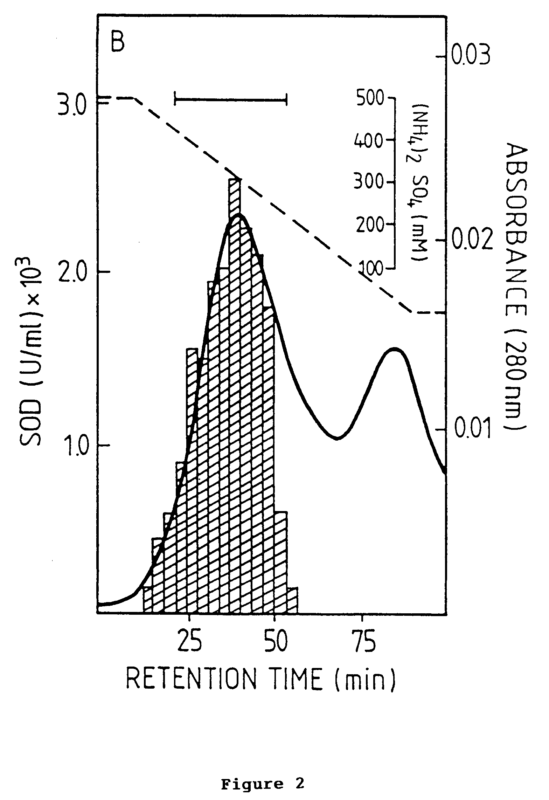

Figure 2: Purification of rat EC-SOD B by means of hybrophobic interaction chromatography on phenyl-Sepharose 4B. The active fractions of the six heparin-Sepharose columns were pooled, dialysed against 500 mM ammoniumsulphate and 50 mM sodiumphosphate (pH 7.0), and applied onto a phenyl-HPLC column in line with a phenyl-Sepharose 4B column. The SOD-activity was recovered from the latter column by elution with a descending gradient of amoniumsulphate (---). Fractions of 3 ml were collected and tested for their SOD-activity. The latter is represented in a block diagram. OD$_{280nm}$ (_). The active pool is indicated by a horizontal bar.

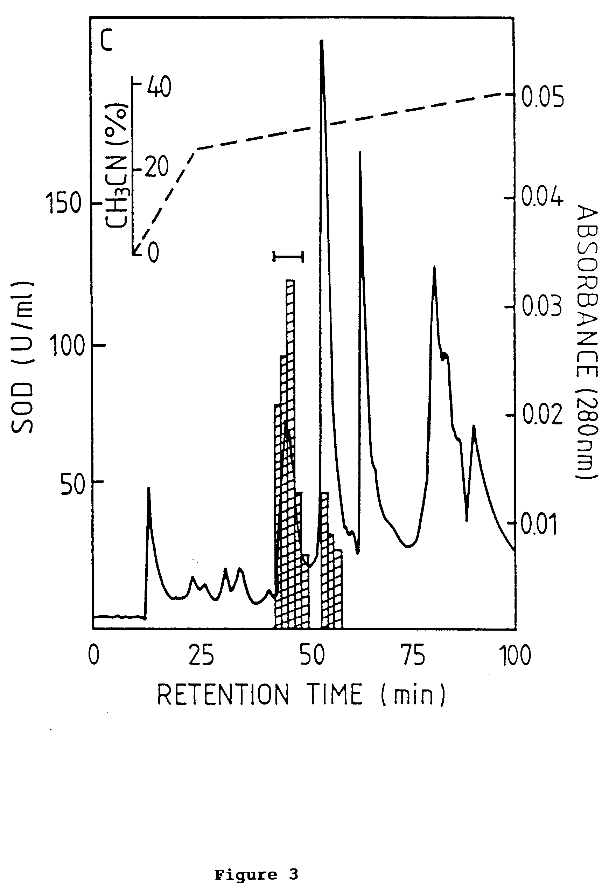

Figure 3: Reversed-phase chromatography on C4. The active pool as purified on phenyl-Sepharose 4B is acidified to 0.1% TFA and applied onto a C4 column. The bound material is eluted with a gradient of acetonitrile (---). Fractions of 1 ml were collected, freeze-dried and tested for their SOD-activity. The activity is represented in a block-diagram. OD$_{280nm}$ (_). The indicated pool is used for amino acid sequence determinations.

Fire 4: Electrophoretic analysis of purified EC-SOD B.
Analysis by 2D-PAGE (pI-gradient, 12.5% AA) under reducing conditions of purified EC-SOD B (silverstaining).

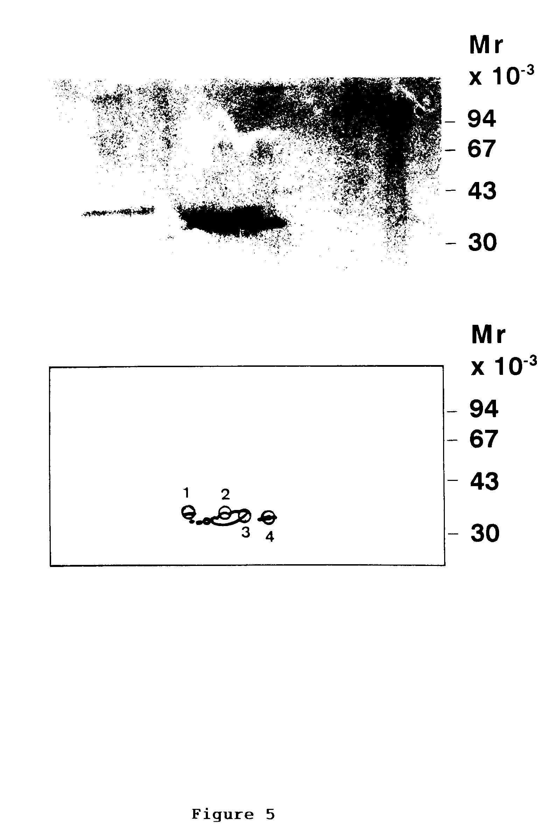

Figure 5: Electrophoretic analysis of purified EC-SOD B.
Analysis by 2D-PAGE (pI-gradient, 12.5% AA) under reducing conditions of purified EC-SOD B (PVDF blotting). The spots marked on the duplicate photograph were subjected to N-terminal amino acid sequence determination (Table 3).

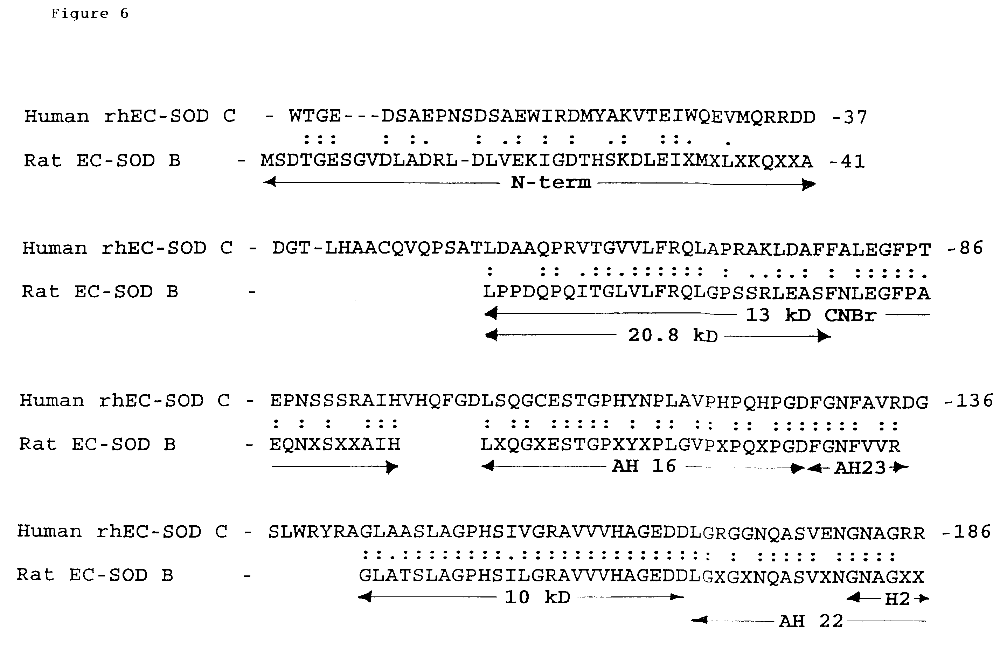

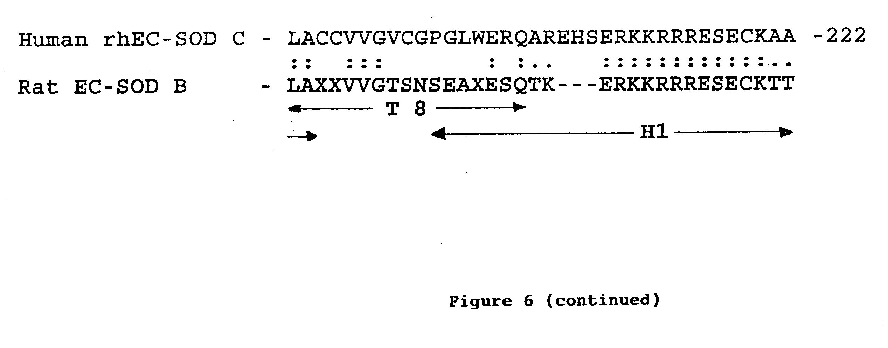

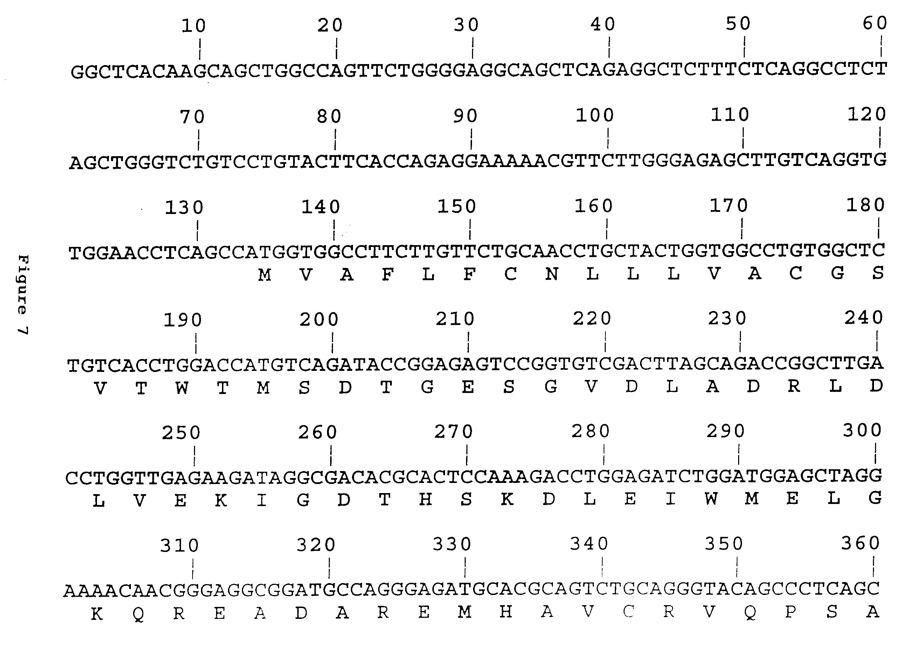

Figure 6: Aligment of rat EC-SOD B peptide sequences generated by partial acid hydrolysis (AH), tryptic digestion (TRYP) and CNBr cleavage (CNBr) with recombinant human EC-SOD C (Hjalmarsson et al., 1987). The amino terminal amino acid sequence of rat EC-SOD was aligned manually with human EC-SOD C (:, sequence identity; ., sequence resemblance). Rat EC-SOD amino acids different from human EC-SOD residues are underlined and in bold. The human EC-SOD C amino acid numbering used in this alignment starts from the start of the signal peptide.

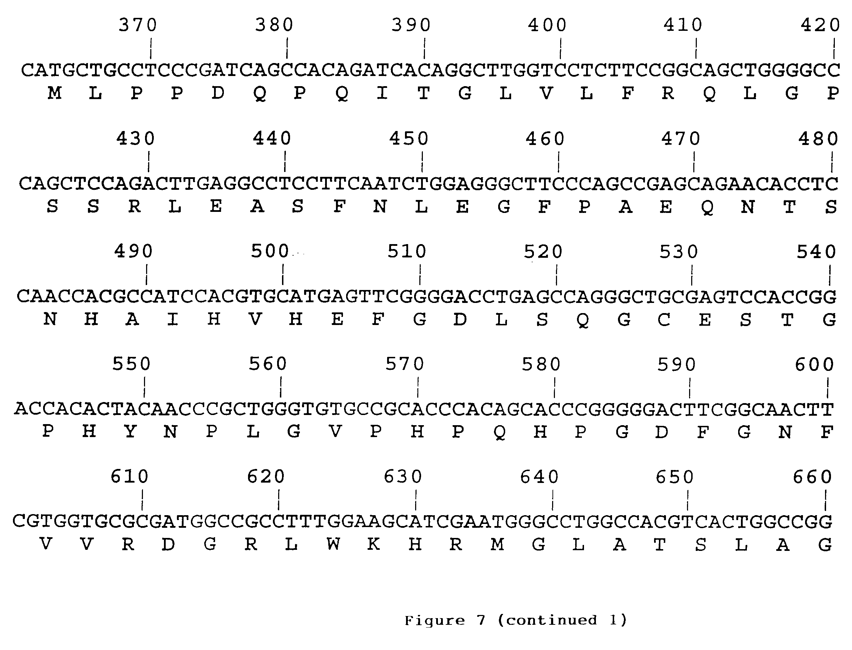

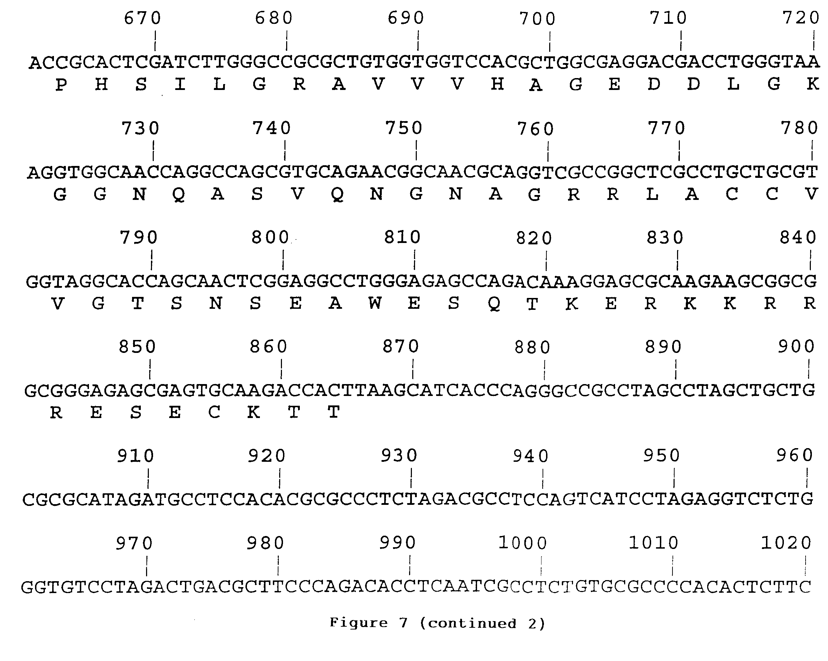

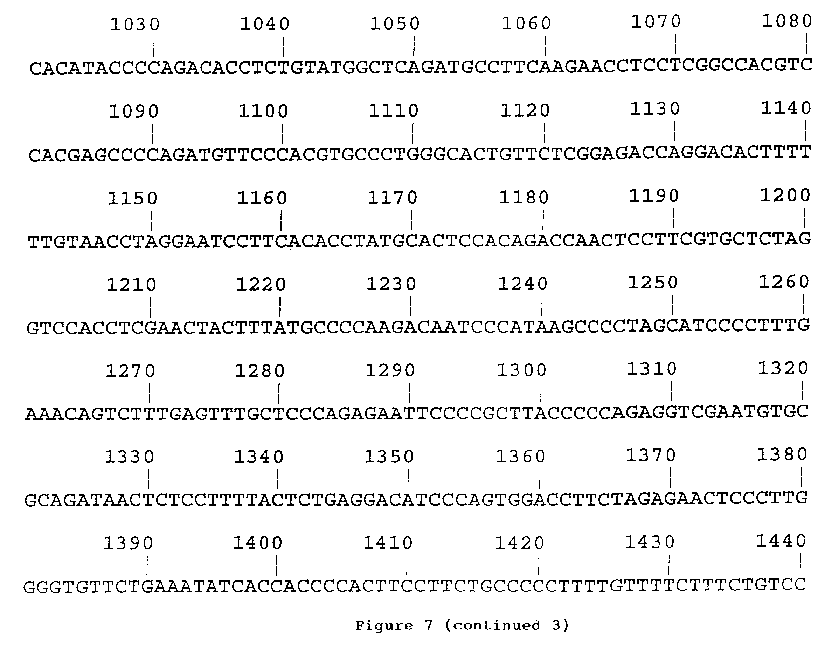

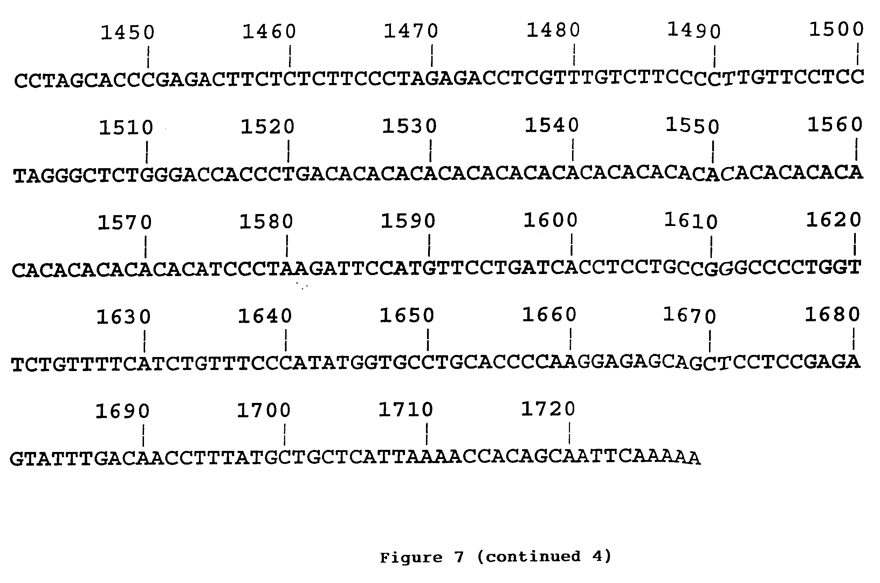

Figure 7: Compilation of nucleotide and amino acid sequences of the complete cDNA sequence of the rat extracellular superoxide dismutase B form. The nucleotide positions are indicated above the nucleotide sequence. The ORF is indicated and the amino acid positions are given under the amino acid sequence.

Figure 8: Alignment of the rat EC-SOD B amino acid sequence (RAECSODB, SEQ ID NO 1) including the signal peptide with the recombinant human EC-SOD C (HUECSODC, Hjalmarsson et al., 1987) including the signal peptide. Sequence identity is indicated by ":", sequence similarity is indicated by ".". Amino acids said to be "similar" are: A,S,T; D,E; N,Q; R,K; I,L,M,V; F,Y,W.

The amino acid position numbers are indicated. The positions of the signal sequence are numbered in a negative way. Both sequences were aligned with a common alignmental algorithm (FASTA). Dashes were introduced in order to maximally align both sequences.

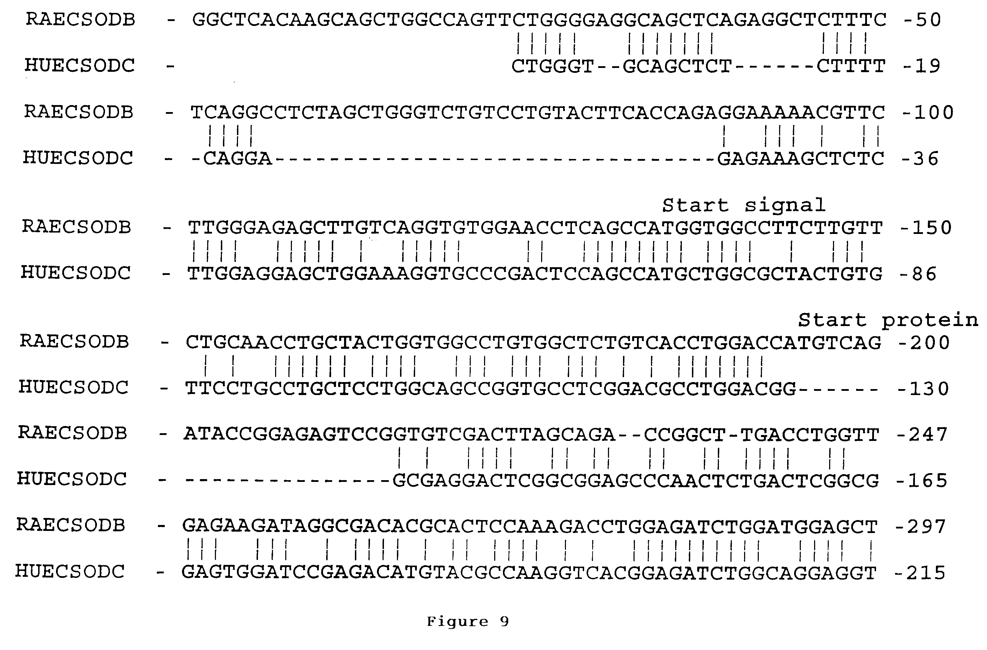

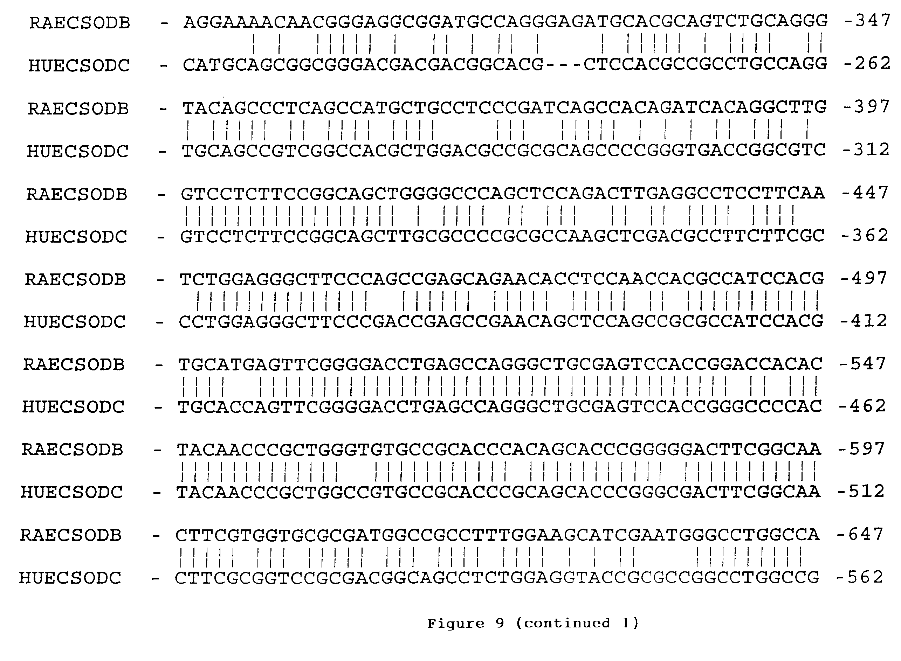

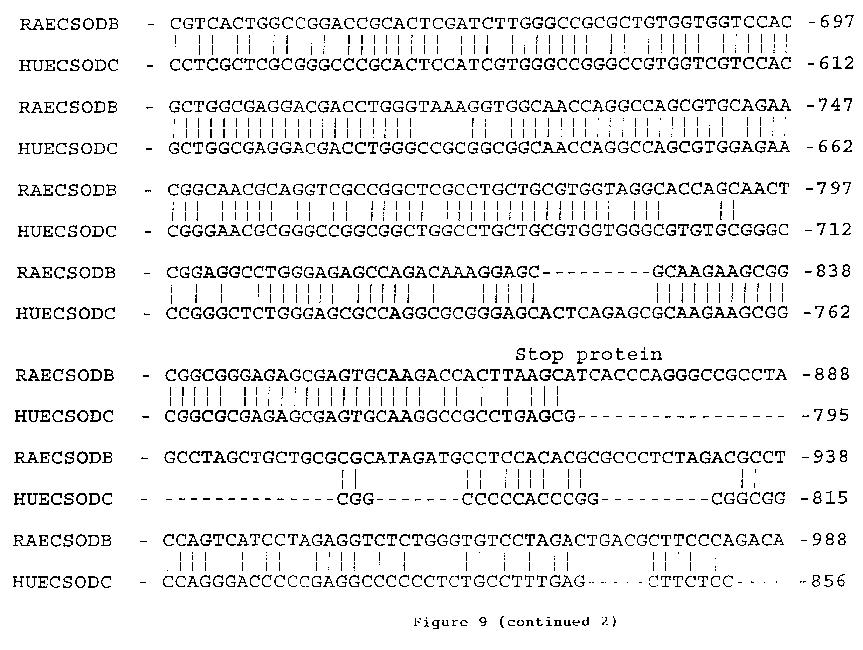

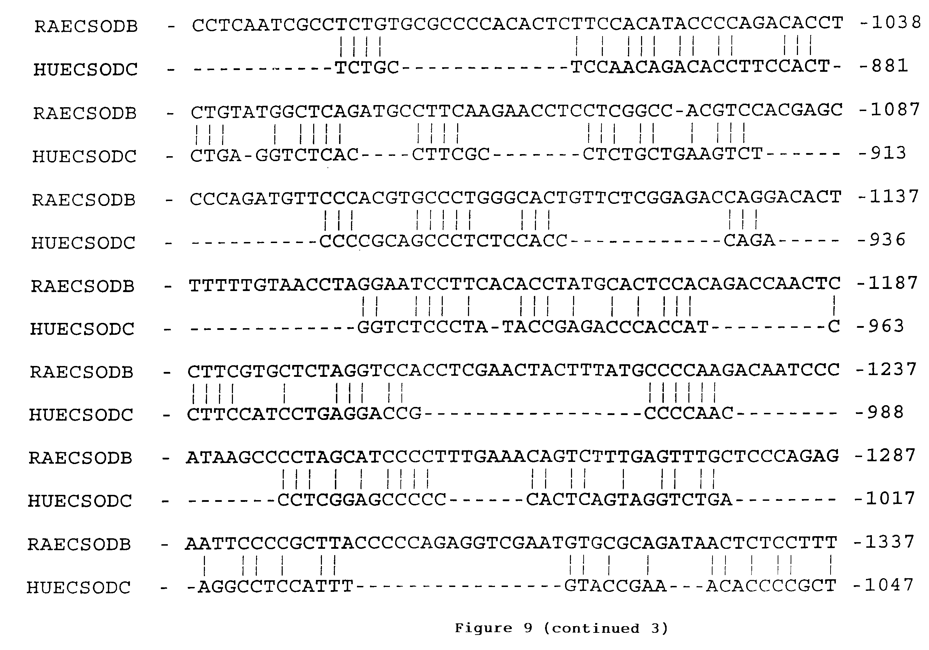

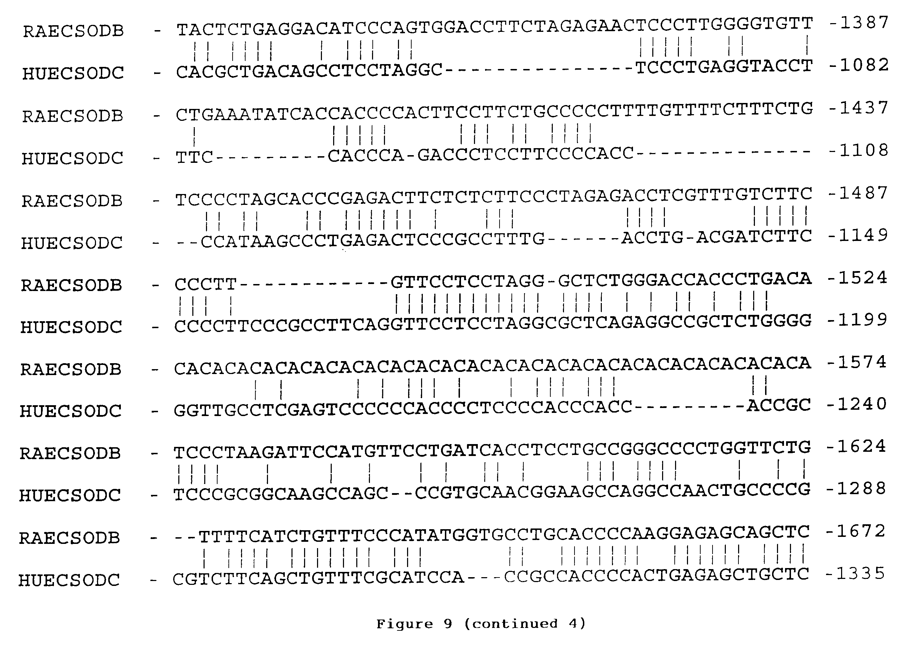

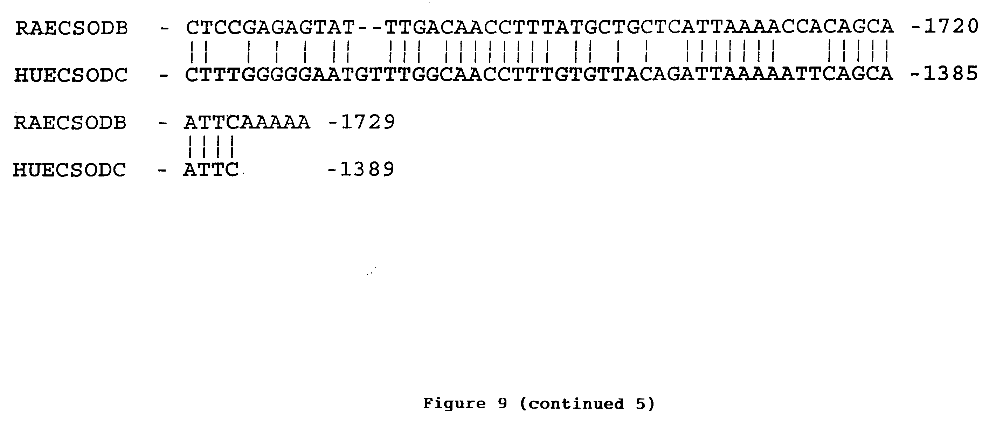

Figure 9: Nucleotide sequence alignment of the rat EC-SOD B form cDNA clone of the present invention (RAECSODB, SEQ ID NO 1) with the human EC-SOD C form (HUECSODC, Hjalmarsson et al., 1987).

## EXAMPLES

### ABBREVIATIONS

CHAPS: 3-(cyclohexylamine)propane sulfonic acid; CFA: complete Freund's adjuvans; CM : conditioned medium; PAGE: polyacrylamide gel electrophoresis; PMA: phorbol 12-myristate 13-acetate; PTH: phenylhydantoine; PVDF: polyvinylidene difluoride; SDS: sodium dodecyl sulphate; SOD : superoxide dismutase; TFA: trifluoroacetic acid; m.c.o. : molecular weight cut-of; RPMI : Roswell Park Memorial Institute medium RPMI 1640 (Gibco, Paisley, Scotland).

### 1. Materials

The rat $C_6$ glioma cell line (ATCC nr. CCL 107) was obtained from Flow Laboratories (Irvine, Scotland). All culture media and additives were from GIBCO - Lifetechnologies (Paisley, Scotland). Plastic culture material was from Becton Dickinson (Oxnard, CA, USA), and the doubletray cell factories from NUNC (Roskilde, Denmark).

Oligo(dT) cellulose was obtained from Collaborative Research (Boston, MA, USA). The multiprime labellingkit was from Amersham (Buckinghamshire, UK), the lambda gt11 rat glioma cDNA library from Clontech (San Francisco USA) and the lambda ZAPII vector from Stratagene (LaJolla, USA).

Secondary antibodies were from Dako (Glostrup, Denmark).

N-glycanase (EC 3.5.1.52) was purchased from Genzyme (Cambridge, MA, USA), Cu-Zn SOD's (from bovine and human erythrocytes) were obtained from Sigma (Brussels, Belgium).

HPLC and FPLC columns were obtained (BIOGEL-HTP hydroxyapatite and a phenyl-HPLC column) from BIO-RAD (Richmond, CA, USA), heparin-Sepharose CL 6B and phenyl-Sepharose (Pharmacia, Uppsala, Sweden), Toyopearl HW 55S and Heparin-HPLC (TSKgel heparin 5PW) from TosoHaas (Montgomeryville, PA, USA), WP-Butyl $C_4$ column (J.T. Bakerbond Research products, Phillipsburg, N.J., USA) and narrow-bore PTC $C_{18}$ (Applied Biosystems, Foster City, CA, USA).

### 2. Cell culture ad conditioned medium (CM)

Rat $C_6$ glioma cells between passage 5 and 15 were cultivated in 175 $cm^2$ flasks in HAM F10 supplemented with 10% FCS, 2 mM L-glutamine, 1% (v/v) MEM vitamins 100x, 1% (v/v) MEM non-essential amino-acids 100x, 100 U/ml penicillin, and 100 $\mu$g/ml streptomycin. Incubation was at 37 °C at 5% $CO_2$ in a humidified atmosphere. At confluence the cells were washed twice with PBS and harvested using 0.025% (w/v) trypsin and 0.01% (w/v) EDTA in PBS. The cells were seeded in serumfree medium at a concentration of approximately 5-6 x $10^4$ cell/$cm^2$ in doubletray cell factories (1,200 $cm^2$). The serumfree medium was HAM F10 : MEM (1:1) (Gibco, Paisley, Scotland) with the above mentioned additives supplemented with 30 nM sodium selenite. The medium is changed after 72 hours and the CM is discarded. The CM is harvested for the first time 96 hours after the medium change, new medium is added and a second harvest of CM is made after a further incubation for 72 hours. Immediately after the removal of residual cells from the CM by centrifugation (1,200 g, 15 min., 4 °C) the latter was applied on a hydroxyapatite column. In a typical preparation approximately 10 L of CM was used as starting material.

### 3. Neutrophil purification

Neutrophils are purified (>97%) from human blood, taken from healthy human volunteers, as described by Willems et al. (1989). They were obtained as viable suspensions in RPMI medium (Gibco, Paisley, Scotland) after dextran sedimentation, centrifugation on Ficoll Hypaque and hypotonic lysis as described previously (Willems et al., 1989).

### 4. Luminescence assay for measuring inhibition of the respiratory burst

Neutrophils (3 x $10^4$ in 150 $\mu$l of RPMI) are incubated together with 150 $\mu$l of RPMI or test sample dilution in RPMI for 90 min at 37 °C. Subsequently 60 $\mu$l of a lucigenin solution ($10^{-3}$ M) is added and the tubes are placed in a Berthold 6 channel Biolumat LB 9505 apparatus (37 °C) to record the background luminescence for 2 min. Finally 60 $\mu$l of PMA (1 $\mu$g/ml) in RPMI is added and luminescence is registrated. The peak values are corrected for background and the inhibition is calculated in relation to the value for tubes without inhibitor (reference tubes). One unit of inhibitory activity is the dilution which gives a 50% reduction of the PMA-induced luminescence value. The 50% reduction is measured from a calibration curve obtained with serial dilutions of a test sample. Using commercial Cu-Zn SOD it was calculated that this assay is about 6 times more sensitive than the classical cytochrome C reduction test (McCord and Fridovich, 1969), which implies that one unit in our assay is equivalent to the activity of approximately 50 ng of Cu-Zn intracellular SOD and 5.8 ng of rat EC-SOD (Table 2). .

## 5. Conditioned medium of $C_6$ inhibits the neutrophil respiratory burst.

CM obtained from rat $C_6$ glioma cells grown in chemically defined medium inhibits the PMA-induced superoxide production measured by the luminescence assay. The extent of the inhibition is maximal in the logaritmic phase of the cell growth (Table 1). However, per cell, the inhibition decreases as a function of the cultivation time.

In order to exclude that the SOD in the CM could be derived from intracellular SOD's as artifacts due to cell lysis, LDH measurements were performed on all CM and were never higher than 3-5% of the total amount of intracellular LDH.

Using other neutrophil activators, such as TNF or arachidonic acid, approximately the same inhibitory values were obtained (data not shown).

TABLE 1

| time | cells/cm$^2$ | U/ml CM | U/10$^4$ cells |
|---|---|---|---|
| 72 hrs | 3.2 x 10$^4$ | 9.9 | 0.61 |
| 144 | 8.5 | 15.8 | 0.37 |
| 216 | 25.8 | 33.0 | 0.21 |
| 288 | 35.0 | 19.8 | 0.11 |
| 360 | 32.6 | 17.6 | 0.11 |

Table 1: Inhibition of the PMA-induced superoxide production as a function of cultivation time. Media conditioned for 72 hours are harvested at the indicated times and the inhibitor U/ml are measured by serial dilution of the CM.

## 6. Purification of the inhibitor of superoxide production

The $C_6$ secreted factor EC-SOD B was purified as outlined in Table 2. The purification includes adsorption chromatography on hydroxyapatite, affinity chromatography on heparin-Sepharose, hydrophobic interaction chromatography (HIC) on phenyl-Sepharose and reversed phase chromatography (RPC) on $C_4$.

With the exception of hydroxyapatite chromatography, all chromatographies were performed at room temperature using a Waters HPLC-system (Millipore, Milford, MA, USA).

### a). Hydroxyapatite chromatography

Hydroxyapatite chromatography was used as a first step in order to concentrate the proteins of approximately 11 L of CM. Each harvest of CM (400 ml/double tray) was diluted (2:1 v/v) with 50 mM Tris/HCl pH 7.5 complemented with 25 $\mu$g/ml fungizone and 100 $\mu$g/ml gentamycin, and applied at 4 °C on a 80 x 50 mm BIOGEL-HTP hydroxyapatite column equilibrated with 10 mM Tris/HCl, 10 $\mu$M CaCl$_2$, pH 7.5 (buffer A). The CM was loaded at a flow rate of 1 ml/min, followed by an extensive wash with buffer A. Discontinuous elution with 500 mM sodium phosphate (pH 7.5) at 20°C concentrated the proteins and increases the specific activity 1.8-fold.

### b). Heparin-Sepharose chromatography

The hydroxyapatite-eluted material was dialysed (m.c.o. 3,500) against 50 mM Tris/HCl (pH 7.1), divided into six equal parts and loaded onto a 210 x 10 mm column of heparin-Sepharose CL 6B, equilibrated with 50 mM Tris/HCl (pH 7.1). The bound material was eluted in 80 min with a linear gradient of NaCl (0-2M) in equilibration buffer.

Heparin-Sepharose affinity chromatography of the hydroxyapatite-eluted fraction resulted in a further 6 to 7 10-fold enrichment of the activity in fractions eluting between 300 and 400 mM NaCl (Figure 1).

### c). Hydrophobic interaction chromatography (HIC)

Active fractions eluted from six heparin-Sepharose CL 6B columns were pooled and dialysed (m.c.o. 3,500) against 500 mM $(NH_4)_2SO_4$, 50 mM sodiumphosphate pH 7.0 and applied onto a phenyl-HPLC column (75 x 7.5 man) on line with a phenyl-Sepharose column (65 x 10 man) both equilibrated with the binding buffer. The bioactivity was not retained on the first phenyl-HPLC column, but was bound on the phenyl-Sepharose column. A fast and easy 10-fold enrichment was obtained in this step. Only, 3 % of the applied protein was retained on the phenyl HPLC column. The activity was retained on the phenyl-Sepharose column and was eluted using a 30 min linear $(NH_4)_2SO_4$-gradient in 50 mM phosphate pH 7.0. (Figure 2). HIC reduced the total bioactivity to 15% (Table 2).

### d). Reversed-phase chromatography

The active fractions eluted from the phenyl-Sepharose column were pooled and dialysed (m.c.o. 10,000) against 20 mM Tris/HCl (pH 7.0). Immediately before application on a Bakerbond $C_4$ reversed phase column (250 x 4.6 mm), the sample was acidified with 0.1% TFA. The applied proteins were eluted in 85 min with a linear gradient from 0.1% TFA, 25% acetonitrile in water to 0.1% TFA in 60% acetonitrile (Figure 3). The remaining activity eluted at approximately 30% acetonitrile. The acidic conditions further lowered the total bioactivity to 0.1% of the initial value and reduced the specific activity to 6,000 U/mg (Table 2). Almost no loss in EC-SOD protein was measured by radioimmunoassays. From the purification scheme it could be estimated that 1 U, which neutralizes 50% of the $O_2-$ produced by PMA activated neutrophils, corresponds to 5.8 ng of rat EC-SOD B.

Table 2

| Purification of rat $C_6$ EC-SOD B | | | | | | | |
|---|---|---|---|---|---|---|---|
| | volume (ml) | prot.conc. (mg) | Units (U/ml) | Units total | spec.act. (U/mg) | SOD* (mg) | ng SOD/U |
| 1. $C_6$ CM | 11,600 | 86.6 | 25 | 290,000 | 3,350 | nd | - |
| 2. Hydroxyapatite | 1,100 | 60.9 | 350 | 385,000 | 6,300 | 2.33 | 6.1 |
| 3. Heparin-Sepharose | 49.0 | 6.1 | 8,000 | 392,000 | 64,300 | 2.30 | 5.85 |
| 4. Phenyl-Sepharose | 32.5 | 0.42 | 1,910 | 62,000 | 147,800 | 0.36 | 5.7 |
| 5. $C_4$-reverse phase | 4.0 | 0.05 | 75 | 300 | 6,000 | 0.05 | - |
| nd = not determined | | | | | | | |

Samples were dialysed against 50 mM Tris/HCl (pH 7.5) before measurement of the SOD-activity and the protein concentration. The latter is determined using BSA as a standard.

### 7. Protein electrophoresis and staining

One-dimensional SDS-PAGE was carried out in a Tris/glycine or in a Tris/tricine buffer system (Laemmli, 1970; Schägger & von Jagow, 1987). Samples were dissolved in sample buffer containing 1% SDS-and 50 mM DTT and heated at 80 °C for 5 min prior to electrophoresis.

Two-dimensional PAGE was carried out as described by O'Farrell (1975).

Analysis of the purified fractions by SDS-PAGE indicated the presence of two polypeptide bands with a molecular mass of 34,000 to 36,000 in the active fractions. Both polypeptides are posttranslational modifications of the same protein as proven by subsequent 2D-analysis and amino acid sequence

* The amount of SOD was estimated from radioimmunoassays. Purified rat EC-SOD was used to calculate the absolute amount of the enzyme after each purification step.

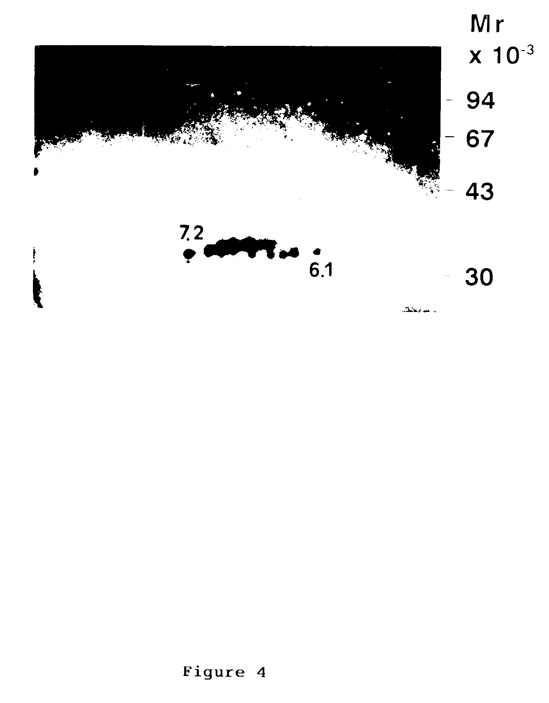

determination. The protein was more than 95% pure. 2D-electrophoresis under reducing conditions resolved the latter protein into two polypeptides present as multiple isoforms (Figures 4 and 5). The two chains of multiple protein spots varied in pI from 6.1 to 7.2 and in molecular mass from 33,000 to 37,000, suggesting extensive post-translational modifications by glycosylation of the two polypeptides. The 2D-gel pattern is characteristic for glycosylated proteins.

## 8. Electroblotting and staining

Electroblotting of proteins onto ProBlott PVDF membranes was done in the presence of 10 mM CAPS as described by Matsudaira (1987). Proteins were visualized by staining with Coomassie brilliant blue R-250, Coomassie brilliant G-250, Ponceau S or silverstaining. Proteins were visualized on the PVDF-blots by staining with Coomassie brilliant blue R-250 or amidoblack. Proteins for amino acid sequencing were carboxymethylated according to Hirs (1969). Electrophoresis, blotting and staining were carried out in the presence of 1 mM thioglycollic acid.

## 9. Amino acid sequence determination of EC-SOD B

Purified peptides were sequenced using a pulse-liquid Model 477A sequenator equipped with an on line 120 phenylthiohydantoin (PTH) analyzer (Applied Biosystems).

In the case of N-terminal sequencing of PVDF bound samples, the excised band or 2D-spots were sliced (2 x 2 mm) and placed in the reaction cartridge on top of a cartridge filter so that an optimal flow of reagents was achieved.

### 9.1. Amino-terminal sequence determination

N-terminal amino acid sequence determinations of four individual polypeptides, separated by 2D-gelelectrophoresis and subsequently blotted on PVDF (Figure 5), revealed identical N-terminal amino acid sequences. This confirms that the different clusters of isoforms all belong to EC-SOD B, and are caused by extensive post-translational modifications. The amino acid sequence was determined for the four distinct proteinspots on the blot (Figure 5) are shown in Table 3. These results confirm that the different protein clusters of protein spots are derived from the same protein by posttranslational modifications. Moreover, the same N-terminal sequence was obtained for the purified enzyme subjected to sequence analysis without prior separation by 2D-PAGE. No additional sequences were detected in this protein sample. The N-terminal sequence could not be aligned with a common aligment algorithm to any protein in the databanks (PIR, Swissprot, Genbank).

Further sequence data were obtained with polypeptides generated by limited acid hydrolysis, CNBr-cleavage, tryptic digestion and hydroxylamine-cleavage (see below).

### 9.2. Limited acid hydrolysis and peptide mapping

Partial acid hydrolysis of proteins immobilized in polyacrylamide gels was based on the method of Vanfleteren et al. (1992). Briefly, Ponceau S stained gel slices were destained with 2% HCOOH. Complete destaining was achieved with 1 mM NaOH (2 x 1.5 ml). One ml of 2% HCOOH was added to the gel slices for 10 min at room temperature. Followed by incubation for 6 hours at 117 °C. The cleavage was stopped by addition of 100 $\mu$l acetonitrile and freezing of the peptide samples.

The EC-SOD derived peptides were separated on a narrow-bore PTC $C_{18}$ column (2.1 x 220 man, Brownlee, USA) by a gradient from 0.1% TFA in water (A) to 0.09% TFA in 70% acetonitrile (B) (in 6 min to 15% B, 6-55 min to 60% B, 55-70 min to 100% B). The column outlet was directly connected to a 8 $\mu$l flow cell of a 1000 S Diode Array detector (Applied Biosystems) and monitored by UV absorbance at 214 nm. Eluting peaks were collected manually and stored at -20 °C until further use. Table 3 gives an overview of the generated peptides.

### 9.3. Cyanogen bromide cleavage and peptide separation

CNBr cleaved peptides were generated by a modification of the method described by Gross (1967). Briefly the reaction is performed in the dark for 24 hours at 30 °C in 70% (v/v) formic acid in water with a 50- or a 200-fold molar CNBr excess over methionine residues. The reaction is stopped by dilution of the sample with 15 vol of water and freeze-drying.

14

The peptides were separated by SDS-PAGE according to the method of Schägger & Von Jagow (1987). Table 3 gives an overview of the generated peptides

### 9.4. Tryptic digestion id peptide mapping

Tryptic digestion of blotted EC-SOD B form was based on the method of Bauw, et al. (1989). Briefly, stained EC-SOD B containing PVDF membrane was cut in pieces (2 mm x 2 mm) and treated with 500 $\mu$l of 0.5 % PVP-40 in methanol. After 30 min. the quenching mixture was discarded and the membrane pieces washed four times with 500 $\mu$l water and once with 500 $\mu$l 100 mM TRIS-Cl pH 8.56 containing 10 % (v/v) acetonitrile. Then, the buffer was replaced with 80 $\mu$l of the same buffer containing 2 $\mu$l of a trypsin solution at 1 mg/ml in 100 mM TRIS-Cl pH 8.56. The digestion was done for 18 hr at 37 °C. After the digestion the PVDF membrane pieces were washed once with 50 $\mu$l 80% formic acid, once with 50 $\mu$l 40 % propionic acid containing 20 % formic acid and once with 100 $\mu$l acetonitrile. All the supernatants were collected in one Eppendorf tube ,vacuum-dried and stored at -20 °C untill HPLC analysis.

The EC-SOD derived tryptic peptides were resolubilised in 10 % acetonitrile containing 0.1 % TFA and separated on a C4 VYDAC column (2.1 mm x 250 mm, Hesperia , CA , USA) using the 140 B Solvent Delivery System (Applied Biosystems). Elution of the peptides was achieved with a linearly increasing gradient of 0.1 % TFA in water (A) to 70% acetonitrile in 0.1 % TFA (B) (equilibration 8 min to 10% B, 8-68 min to 70% B, 68-73 min to 100% B. The column outlet was directly connected to a 8 $\mu$l flow cell of a 1000 S Diode Array detector (Applied Biosystems) and monitored by UV absorbance at 214 nm. Eluting peaks were collected manually and stored at -20 °C untill further use.

A large peak eluting at 40 % acetonitrile is derived from remaining quantities of PVP-40. No peak collection could be performed in this region. The peptides were loaded on a precycled biobrene cartridge filter and sequenced using the standard NORMAL-1 sequencer program without modifications. Table 3 gives an overview of the generated peptides.

### 9.5. Hydroxylamine-generated peptides

EC-SOD was reduced and carboxylmethylated with iodoacetamide as described by Hirs (1969) prior to hydroxylamine hydrolysis according to Adachi et al. (1992). The reaction mixture contained 1.5 M hydroxylamine/HCl, 0.15 M $K_2CO_3$ and 4.6 M guanidinium/HCl. Table 3 gives an overview of the obtained amino acid sequences.

## <u>Table 3</u>. Determined amino acid sequences of rat EC-SOD B

| | Corresponding human |
|---|---|
| * N-terminal sequence | EC-SOD C residues |
| 2D-blot spot 1: [1]MSDTGESGVDLADXLXLVEKI[21] ... (SEQ ID NO 7) | - |
| spot 2: [1]MSDTGESGVDLADRLDLVEKIGDTHSKDLEIXMXLXKQXXA[41] ... (SEQ ID NO 8) | - |
| spot 3: [1]MSDTGES[7] ... (SEQ ID NO 9) | - |
| spot 4: [1]MSDTGESGVDLADRLDLVEK[20] ... (SEQ ID NO 10) | - |
| * CNBr fragments | |
| 20.8 kD: (M) [58]LPPDQPQITGLVLFRQLGPSXRLEA[82] ... (SEQ ID NO 11) | L[53] - A[77] |
| 13 kD: (M) [58]LPPDQPQITGLVLFRQLGPSSRLEASFNLEGFPAEQNXSXXAIH[101]...(SEQ ID NO 12) | L[53] - H[96] |
| 10 kD: (M)[149]GLATSLAGPHSILGRAVVVXAXEDD[173]... (SEQ ID NO 13) | G[144] - D[168] |
| * Tryptic digest | |
| T8: (R)[192]LAXXVVGTSNSEAXESQ[208]... (SEQ ID NO 14) | L[187] - Q[203] |
| * Acidic hydrolysis | |
| AH 23: (D)[133]FGNFVVR[139] (SEQ ID NO 15) | F[128] - R[134] |
| AH 16: (D)[108]LXQGXESTGPXYXPLGVPXPQXPGD[132] (SEQ ID NO 16) | L[103] - D[127] |
| AH 22: (D)[174]LGXGXNQASVXNGNAGXXLA[193]... (SEQ ID NO 17) | L[169] - A[188] |
| * Hydroxylamine cleavage | |
| H1: (N)[202]SEAWESQTKERKKRRRESECKTT[224] (SEQ ID NO 18) | P[197] - A[222] |
| H2: (N)[186]GNAG[188]... (SEQ ID NO 19) | G[181] - G[184] |

The determined amino acid sequences could be aligned with those present in data banks (SWISS-PROT, PIR). An alignment with human EC-SOD C using the FASTA program (Pearson and Lipman, 1988) is shown in Figure 6.

The hydroxylamine-cleavage product H1 contained the carboxylterminal sequence of the purified enzyme.

The CNBr-generated fragment of 10,000 dalton could be aligned with a sequence located in the active site of SOD and is generated by a cleavage carboxyterminal of Met148 in the rat EC-SOD sequence (Fig. 6). The CNBr-fragments of 20,800 dalton and 13,000 dalton were generated by cleavage carboxyterminal of Met57. The later sequences have a low homology with the human EC-SOD C sequence.

Except for the N-terminal sequence all sequence data could be aligned with recombinant human EC-SOD C from Leu53 up to the carboxyterminus. More than 90% of the EC-SOD sequence is determined by amino acid sequence (Fig. 6). Several other polypeptides, generated by acidic hydrolysis and separated by reverse phase, have been sequenced. The latter are already included in the sequences of AH 16, AH 22 and AH 23 and support the existence of one protein with a complex 2D-pattern. The sequence data identify the enzyme as a rat homologue of the human EC-SOD. As the rat does not contain the C-form of EC-SOD the purified enzyme has to be the EC-SOD B form (Karlsson & Marklund, 1988).

## 10. Isolation of the full-length rat EC-SOD B cDNA

### 10.1. Synthesis of a rat C6 glioma cDNA library

A rat C6 λgt11 cDNA library cDNA is commercially available from Clontech (San Francisco, CA, USA) or can be prepared in the λZap II vector (Stratagene) starting from poly-(A)$^+$ RNA purified from the rat C6 glioma cell line using the following procedure.

### 10.1.1. Preparation of mRNA from rat C6-glioma cells

Total RNA was isolated by the acid-guanidinium-phenol method as described by Chomcsynski and Saachi (1983). Poly-(A)$^+$RNA was purified by oligo(dT)-cellulose chromatography (Type 3; Collaborative Research, Boston, MA, USA) as described by Aviv and Leder (1972).

### 10.1.2. First-strand cDNA synthesis

Reaction mixtures (25 $\mu$l) contained 50 mM Tris-HCl (pH 8.3), 75 mM KCl, 10 mM DTT, 3 mM MgCl$_2$, 20 $\mu$g/ml of hexameric oligonucleotides of randomized sequence (Pharmacia) or 50 $\mu$g/ml of oligo(dT)12-15, 50-200 $\mu$g/ml of poly A$^+$ RNA and 5,000-10,000 units/ml of superscript reverse transcriptase. After the addition of enzyme, a 5-$\mu$l aliquot was transferred to a separate tube containing 0.5 $\mu$l of $\alpha$($^{32}$P)-dCTP (1,37.10$^4$ - 1,37.10$^5$ Bq)). Both tubes were incubated for one hour at 37°C. The tube containing 5 $\mu$l was processed to determine the yield of cDNA and to analyze the cDNA by alkaline agarose gel electrophoresis.

### 10.2. Polymerase chain reaction (PCR) to generate a rat EC-SOD B cDNA probe

### 10.2.1.Design of PCR primers

Based on the partial amino acid sequence of the rat EC-SOD B from (see Table 4) and the alignment to human EC-SOD C (Figure 6), two degenerate primers were synthesized and used to generate the first cDNA strand. Oligonucleotide synthesis was carried out by the phosphoramidite method using an Applied Biosystems DNA synthesizer Model 392 (Applied Biosystems, Foster City, CA, USA).

The antisense primer I relates to the amino acid sequence "DFGNFV" of rat EC-SOD B which correspond to positions 127 to 131 of human EC-SOD C as shown in Fig. 6. This sequence is a combination of position 25 of SEQ ID NO 16 and positions 1 to 5 of SEQ ID NO 15 as shown in Table 3.

PRIMER I 5' IACRAARTTICCRAARTC 3' (SEQ ID NO 3)

The sense Primer II relates to the amino acid sequence "PDQPQI" of as shown in Fig. 6. This sequence contains positions 3 to 8 of SEQ ID NO 12 as shown in Table 3.

PRIMER II 5' CCIGAYCARCCICARATHAC 3' (SEQ ID NO 4)

### 10.2.2. Generation of the rat EC-SOD cDNA probe by PCR

First strand cDNA synthesis was carried out as described in section 10.1. Standard PCR reactions were performed in a final volume of 50$\mu$l containing 1$\mu$l of cDNA, 10 to 50 pmoles of deoxynucleotide primer, 200$\mu$M dNTPs, 50 mM KCl, 10mM Tris-Cl (pH 13.3), 1.5 mM MgCl$_2$, 0.001% gelatin and 1.2 units of Taq polymerase (Stratagene). Samples were overlaid with paraffin oil. After denaturation for 5 minutes at 95°C, routinely 30 cycles were performed using cycles of 1 minute at annealing temperatures (between 37 to 50°C), 2 minutes at 72°C and 30 seconds at 95°C).

As the degenerate primers have variable melting temperatures, different annealing temperatures (37 to 50°C) were tested to obtain the EC-SOD B amplification fragment. For the given primer combinations a PCR amplicon with an estimated length of 250 bp was generated at the annealing temperature of 37°C.

Preparative amounts of these PCR fragments were generated by a second PCR amplification round, starting from 1/10 (or 5 $\mu$l) of the first PCR material using the same conditions as described above.

Subsequently the DNA was purified by the method described by Zhu et al. (1986). The purified DNA was filled in with Klenow polymerase and cloned into EcoRV-cut Bluescript SK+plasmid. Plasmid DNAs from 18 clones was prepared by Qiagen protocol. DNA sequencing confirmed that a selected clone contained the sequence that matched perfectly with the Rat EC-SOD B amino acid sequence comprising Asp (61) to Val (137).

### 10.3 Screening of the C6 rat glioma cDNA library for the full size rat EC-SOD cDNA

Based on the nucleotide sequence of the above-mentioned 250 bp fragment, a

58-bp                                                                                  probe
(5'GTCTGGAGCTGGGCCCCAGCTGCCGGAAGAGGACCAAGCCTGTGATCT
GCGGCTGATC 3)' (SEQ ID NO 5)

was synthesized using the Applied Biosystems DNA Synthesizer Model 392 (Applied Biosystems, Foster City, CA). This 58-bp probe was used for screening the lambda gt11 library of rat C6 glioma for full-length cDNA clones. The probe was radioactively labelled with $^{32}$P-ATP by T4-polynucleotide kinase (Boehringer) and separated from unincorporated nucleotides by Sephadex G-50 chromatography.

**10.3.1 Primary screening**

The C6 λgt11 cDNA library was plated at a high density (about $10^5$ plaques/400 cm$^2$ plate) on LB agar plates and lifted to duplicate nylon membranes essentially as described by the maunfacturer (Amersham, UK).

Prehybridization was carried out either overnight or for 4 hours in 20% formamide, 5X SSPE buffer, 0.1% SDS, 0.1% milk powder, 100 $\mu$g/ml of Herring sperm DNA at 40°C. Hybridization was carried out overnight in prehybridization solution containing 50 $\mu$g/ml of E.coli tRNA and $10^6$ cpm/ml of radioactively labelled probe. The filters were washed sequentially with 6 X SSC/0.1% SDS and 2 X SSC/ 0.1% SDS at room temperature and finally at 42°C in 2 X SSC/0.1% SDS. These filters were autoradiographed at -70°C using intensifying screens. Positive plaques located at identical positions on duplicate filters were picked up and used for secondary screening.

**10.3.2 Secondary screening**

Phages from the primary screening were plated out at lower density, plaque lifted onto nylon membranes and baked at 80°C for 2h. Prehybridisation, hybridization and washing conditions are similar to that described for primary screening. The plaques hybridizing with the 250 bp partial cDNA clone labeled with the $\alpha$$^{32}$P-CTP by Multi prime labelling were subjected to another round of screening with a 250 bp probe. Individual plaques hybridizing with the probe were isolated in order to prepare DNA for subcloning and sequencing.

**10.3.3 Subcloning and sequencing**

From the purified plaques, lambda DNA was prepared by DEAE-cellulose method as essentially described by Zagursky et al. (1985). As the cDNA inserts were difficult to excise using the restriction enzyme EcoRI, KpnI and SacI enzymes were used to excise the inserts from lambda DNA which were subcloned into Bluescript SK(+) plasmid cut with KpnI and SacI. Alternatively the cDNA inserts were prepared by PCR amplification using lambda gtll specific primers (Clontech). DNA sequencing carried out on the PCR amplified products extended the sequence information of Rat EC-SOD cDNA towards 5' and 3' direction. Bluescript subclones carrying SacI/StuI fragment and a EcoRI/SmaI fragment were prepared from gtll EC-SOD clone and subjected to DNA sequence analysis. Out of 7.5 x $10^5$ plaques, three positive clones were obtained. However, only 1 clone contained an SOD sequence from nucleotide position 269 to position 789 of Figure 7. The cDNA isolated from the library still lacked the information at the 5'end. Based on the latter sequence antisense and sense primers were synthesized to extend the sequence towards the 5' and the 3' ends.

**10.3.4 5' ends of the rat EC-SOD B cDNA**

In order to obtain the 5'ends of the rat EC-SOD B cDNA obtained as described above, ligase anchored PCR was carried out as described by Trout et al. (1992). Briefly, a synthetic oligo having the sequence 5'TTTAGTGAGGGTTAATAAGCGGCCGCGTCGTGACTGGGAGCGC 3' was ligated to the 3'ends of first strand cDNA prepared from poly (A)$^+$RNA of rat C6 glioma cells. After ligation, PCR was carried out using a rat EC-SOD specific primer corresponding to nucleotides 335 to 354 (SEQ ID NO 6) and a primer complementary to a part of synthetic oligo (5'GCGGCCGCTTATTAACCCTCACTAAA 3'). Reactions were carried out as follows : cycle 1,5 min. at 94°C, 60 sec. at 55°C and 90 sec. at 70°C; cycles 2-40, 45 sec. at 94°C, 60 sec. at 55°C and 90 sec. at 70°C. The PCR products were separated on agarose gel, transferred to Hybond N+ membranes and hybridized to $P^{32}$ labelled Bgl II/Stu I fragment of rat EC-SOD cDNA. A 400 bp fragment obtained from PCR was filled in with klenow polymerase, subcloned into EcoRV-cut Bluescript SK(+) plasmid and sequenced.

Sequence analysis revealed two point mutations in the 400 bp fragment (based on comparison to amino acid sequence) on either side of Sal I site (position 218). Using two clones, each of them carrying one mutation at different site and the cDNA isolated from library, a full length cDNA was constructed. The nucleotide sequence obtained was 1729 basepairs long and contained an open reading frame of 224 amino acids flanked by 133 basepairs in the 5' and 863 basepairs in the 3' untranslated regions. This complete cDNA sequence of rat EC-SOD B is given in SEQ ID NO 1. Nucleotide sequence and deduced amino sequence are given in Figure 7. Figure 8 shows an alignment of the rat EC-SOD B amino acid sequence with the human EC-SOD C sequence. Figure 9 gives an alignment of the rat EC-SOD B nucleotide sequence with the human EC-SOD C sequence.

The human and rat EC-SODs are very homologous in regions which align with the intracellular SODs. Those regions mainly comprise the active site of the enzymes.

Except for two residue, all invariant amino acids of the intracellular SODs can be identified in the primary structure of their extracellular counterparts (Table 4). The hydrophilic C-terminal region, which has been proposed as the heparin-binding site of human EC-SOD C and the consensus sequence for glycosylation at Asn (94) are also conserved in the rat homologue.

The main differences in the primary structure of the human and the rat EC-SOD are observed in regions flanking the active site and the heparin-binding site and comprises residues $Met^1$-$Ile^{60}$, $Thr^{199}$-$Lys^{210}$ and the two carboxyterminal amino acids (Figure 7 and 8). The sequence flanking the carboxyterminal site of the active center lacks the GlyProGly sequence present in human EC-SOD.

Using the FASTA program (Pearson & Lipman, 1988), the rat N-terminal sequence only aligns with human EC-SOD in residues carboxyterminal of Ile (60) are included in the alignment search. Hydrophobicity analysis according to Kyte and Doolittle (1982) further shows that differences only occur in the N-terminal sequence. The latter is more hydrophilic in rat than in human EC-SOD.

The function of the N-terminal sequence of the EC-SODs is still unknown.

## Table 4. Location of the invariant amino acids in rat EC-SOD

Active site residues:

* Metal liganding residues: $His^{101}$, $His^{103}$, $His^{118}$, $His^{126}$, $His^{129}$, $His^{168}$, $Asp^{132}$

* Positioning of $Cu^{2+}$ and $Zn^{2+}$ ligand residues: $Asp^{172}$, $Gly^{131}$

* Structure determinants of the active site: $Gly^{88}$, $Gly^{186}$, $Pro^{121}$

* ß Bulge glycines at opposite ends of the active site channel: $Gly^{116}$, $Gly^{189}$

* Superoxide binding pocket $Arg^{191}$

| | |
|---|---|
| Dimer contacts: | $Gly^{106}$, $Gly^{162}$ |
| Disulfide bridge: | $Cys^{112}$, $Cys^{194}$ |
| Greek key ß barrel fold: | $Phe^{89}$, $Leu^{154}$ |

The invariant amino acids (Bannister et al., 1987; Getzoff et al., 1989) have been deduced from the alignment of rat and human EC-SOD with the intracellular SODs. Invariant amino acid $Gly^{16}$ and $Gly^{147}$ are mutated to $Pro^{63}$ and $Cys^{195}$ in the extracellular SOD respectively.

## 11. Cloning of Human EC-SOD B cDNA

A lamda gt 11 library derived from mRNA of human WI-38 cells was plated out at a high density ($10^5$ plaques/400 $cm^2$ plate) on LB agar plates and lifted into duplicate nylon membranes essentially as described by the manufacturer (Amersham, U.K.).

Prehybridization was carried out either overnight or for 4 hours in 6xSSC/2x Denhardts solution/5x SSPE buffer and 0.1 % SDS.

The library was screened with a BglII/SmaI fragment of rat EC-SOD cDNA multi-prime labelled to a high specific activity with $\alpha^{32}$P-CTP. Hybridization was carried out overnight in prehybridization buffer containing

$10^6$ cpm/ml of the labelled probe. The filters were washed sequentially with 6xSSC/0.1 % SDS and 2xSSC/0.1 % SDS at room temperature and finally with 2xSSC/0.1 % SDS at 45°C. The filters were autoradiographed at -70°C with intensifying screens. Positive plaques located at identical positions on duplicate filters were picked up and subjected to another two rounds of hybridization with Bgl II/Stu I fragment of the Rat EC-SOD cDNA under the same conditions described for primary screening.

From the purified plaques, lamda DNA was prepared by DEAE cellulose method as essentially described by Zagursky et al (1989).

## 12. Radioimmunoassay and ELISA for EC-SOD

A rabbit was primed and boosted twice with 15 to 20 $\mu$g of purified EC-SOD B together with CFA. From about 100 ml of antiserum an IgG fraction was obtained by $Na_2SO_4$ precipitation (39.1 mg/ml).

Briefly, the radioimmunoassay for EC-SOD B was performed as follows: cups of a microtiter plate were coated with 50 $\mu$l of SOD containing fractions, postcoated with BSA (2 mg/ml in PBS), incubated with 50 $\mu$l of the rabbit IgG anti EC-SOD B (1:250 in PBS), washed three times (PBS containing 0.2% Tween 80), incubated with $^{125}$I labelled Sheep anti-rabbit IgG antibodies, washed and counted in a Packard Gamma counter.

Under these conditions less than 1 ng of B form can be detected. Cross reactivity of our antiserum with $Cu^{2+}$-$Zn^{2+}$ SOD is about 1.5%.

ELISA was performed as follows: wells were coated with 50 $\mu$l fractions, postcoated with 10% saturated casein in PBS (blocker), incubated with rabbit IgG anti EC-SOD B (1:700 in blocker), washed three times with 0.05% Tween 20 in PBS, incubated with peroxidase labelled anti-rabbit IgG antibodies (1:3000 in blocker) and washed. A TMB-staining was used to visualise positive signals.

## 13. Binding to neutrophils

The affinity of EC-SOD B for neutrophils was measured with SOD purified from phenyl-Sepharose chromatography bioactive pool by chromatography on a TSK gel heparin 5PW. Since EC-SOD B binds firmly to plastic and glass, assays were performed in gelatin-coated tubes (4 mg/ml) to avoid fixation of EC-SOD B.

Tubes are coated for 1 h. with 3 ml of gelatin solution in PBS (4 mg/ml). They are washed several times with water, and then 1.5 ml Ficoll Paque Solution (Pharmacia, Upsalla, Sweden) is added to the empty tubes. On top of it, 250 $\mu$l granulocyte (neutrophil) suspension (3 x $10^5$/ml in RPMI) plus 50 $\mu$l RPMI (control sample) or SOD in RMPI (100-200 U/ml) are layered. After a 15 min. incubation at room temperature, the tubes are centrifuged for 10 min. at 800 g. During this centrifugation step granulocytes sediment to the bottom. Non-bound EC-SOD B remains on the top zone while bound EC-SOD B cosediments with the neutrophils. After sedimentation, all liquid was removed by careful siphoning starting from the top and proceeding in stepwise fashion to the bottom of the tube. SOD bound to the neutrophils is measured in the neutrophil pellet after addition of 250 $\mu$l of RPMI and incubation for 1 h at 37°C. Finally lucigenin solution (50 $\mu$l, $10^{-3}$ M) is added, background luminescence is noted and then 50 $\mu$l of PMA (1$\mu$g/ml) is added to start the respiratory burst (Berthold 6 channel apparatus). The amount of SOD is estimated from the reduction in luminescence. The results of this neutrophil binding experiment are given in Table 5.

Table 5

| SOD form | Number of Experiments | Luminescence | Probability |
|---|---|---|---|
| Human Cu/Zn SOD (Sigma) | n = 4 | 112 ± 10 | n.s. |
| Bovine Cu/Zn SOD (Sigma) | n = 6 | 96 ± 5 | n.s. |
| EC SOD (HAP column) | n = 5 | 81 ± 14 | p = 0.06 |
| EC SOD (Phenyl Sepharose column) | n = 8 | 74 ± 11 | p = 0.03 |
| EC SOD (Heparin HPLC) | n = 5 | 88 ± 9 | p = 0.06 |

Table 5: results of the neutrophil binding assay. Column 1 depicts the number of experiments. Column 2 depicts the % of PMA-induced luminescence in absence of SOD (set equal to 100%). Column 3 depicts the probability using the non-parametric paired Willcox test. The annotation n.s. means non significant.

## 14. Biochemical characteristics of rat EC-SOD B

Contrary to other SOD's which are acid stable, the purified rat polypeptide of the present invention is acid labile. Its lability was demonstrated by incubation at pH 6.0, 4.0 and 2.0 at 4°C and for 16 h. After readjustment to pH 7.4, the remaining bioactivity was determined. The latter decreased to 55%, 13%, and 8% respectively.

The native molecular mass of EC-SOD was measured by gelfiltration on a Toyopearl HW 55S and on a Superose 12 column (430 x 10 man). The columns were calibrated with ferritin (440,000), aldolase (128,000), transferrin (78,000), BSA (68,000), ovalbuniin (43,000) and chymotrypsinogen A (25,000). The native molecular weight of rat EC-SOD was approximately 85,0000 ± 20,000. The estimated molecular mass indicates a dimeric structure for the rat enzyme.

Protein concentrations were determined using the microprotein assay described by Bradford (1976).

## REFERENCES

Adachi T and Marklund S (1989) Interactions between human extracellular superoxide dismutase C and sulphated polysaccharides. J Biol Chem 264:8537-8541.

Adachi T, Ohta H, Hirano K, Hayashi,K. & Marklund,S.L. (1991) Non-Enzymic Glycation of Human Extracellular Superoxide Dismutase. Biochem J 279:263-267.

Aviv H, Leder P (1972) Purification of biologically active globin mRNA by chromatography on oligothymidic-acid cellulose. Proc Natl Acad Sci (USA) 69:1408-1412.

Bannister J, Bannister W, Rotilio G (1987) Aspects of the Structure, Function, and Application of Superoxide Dismutase. CRC Critical Reviews in Bioch, 22:111-180.

Bauw G, Van Damme J, Puype M, Vandekerckhove J, Gesser B, Ratz G, Lauridsen J, Celis J (1989) Protein elcetroblotting and micro-sequencing strategy in generating protein data bases from two-dimensional gels. Proc Natl Acad Sci (USA) 86:7701-7705.

Becker E (1988) The cytotoxic action of neutrophils on mammalian cells in vitro. Progr Allergy 40:183-208.

Blalock J (1990) Complementarity of peptides specified by 'sense' and 'antisense' strands of DNA. Trends Biotechnolo 8:140-144.

Bradford M (1976) A Rapid and Sensitive Method for the Quantitation of Microgram Quantities of Protein Utilizing the Principle of Protein-Dye Binding. Anal Biochem, 72:249-254

Chomczynski P, Saachi N (1983) Single step methods of RNA isolation by acid guanidinium thiocyanate phenol chiorophorm extraction. Anal Biochem 162:156-159.

Densen P, Mandell G (1985) Granulocytic phagocytes. In: Mandell G (ed.) Principle and practice of infectious diseases. 2nd ed. London Churchill Livingstone Publ: 57-72.

de Préval (1978) Immunoglobulis; In: Bach J Immunology, New York, Wiley and Sons:144-219.

Fleishmann J, Davie J (1984) Immunoglobulins: allotypes and idiotypes. In: Paul W. (ed.) Fundamental Immunology. New York, Raven Press:205-220.

Furakawa K, Tengler R, Nakamura M, Urwyler A, de Weck A, Kanegasaki S, Maly F (1992) B lymphoblasts show oxidase activity in response to cross-linking of surface IgM and HLA-DR. Scand J Immunol 35:561-567.

Gheuens J, McFarlin D (1982) Use of monoclonal anti-idiotypic antibody to P3-X6Ag8 myeloma protein for analysisand purification of B lymphocyte hybridoma products. Eur J Immunol 12:701-703.

Ghiso J, Saball E, Leoni J, Rostagno A, Frangion (1990) Binding of cystatin C to C4: the importance of antisense peptides and their interaction. Proc Natl Acad Sci (USA) 87:1288-1291.

Gross E (1967) The Cyanogen Bromide Reaction. Methods Enzymol, 11:238-255.

Hirs C (1967) Reduction and S-Carboxymethylation of Proteins. Methods Enzymol, 11:199-203.

Hjalmarsson K, Marklund S, Engström A, Edlund T (1987) Isolation of Sequence of Complementary DNA Encoding Human Extra-Cellular Superoxide Dismutase. Proc Natl Acad Sci (USA), 84:6340-6344.

Karlsson K, Marklund S (1987) Heparin-Induced Reslease of Extracellular Superoxide Dismutase to Human Blood Plasma. Biochem J, 242:55-59.

Karlsson K, Marklund S (1988) Extracellular Superoxide Dismutase in the Vascular System of Mammals. Biochem J, 255:223-228.

Karlsson K, Marklund S (1989) Binding of Human Extracellular-Superoxide Dismutase C to Cultured Cell Lines and to Blood Cells. Lab Invest, 60:659-666

Laemmli U (1970) Cleavage of Structural Proteins During the Assembly of the Head of Bacteriophage T4. Nature 227:680-685.

Marklund S (1982) Human Copper-Containing Superoxide Dismutase of High Molecular Weight. Proc Natl Acad Sci (USA), 79:7634-7638.

Marklund S (1984) Extracellular Superoxide Dismutase and Other Superoxide Dismutase Isoenzymes in Tissues from Mammalial Species. Biochem J, 222:649-655.

Marklund S (1990) Expression of Extracellular Superoxide Dismutase by Human Cell Lines. Biochem J, 266:213-219.

Marklund S, Holme E, Hellner L (1982) Superoxide Dismutase in Extracellular Fluids. Clin Chim Acta, 126:41-51.

Matsudaira P (1987) Sequence from Picomole Quantities of Proteins Electroblotted onto Polyvinylidene Difluoride Membranes. J Biol Chem, 262:10035-10038.

McCord J, Fridovich I (1969) Superoxide Dismutase. An Enzyme for Erythrocuprein. J Biol Chem, 244:6049-6055.

O'Farrell P (1975) High Resolution Two-Dimensional Electrophoresis of Proteins. J Biol Chem 250:4007-4021.

Perrson M, Coathien R, Burton D (1991) Generation of diverse high affinity human monoclonal antibodies by repertoire cloning. Proc Natl Acad Sci (USA) 89:2432-2436.

Roubos E (1990) Sense-antisense complementarity of hormone-receptor interaction sites. Trends Biotechnol 8:279-281.

Sambrook J, Fritsch E, Maniatis T (1989) Molecular cloning: A laboratory manual. 2nd ed., Cold Spring Harbor Laboratory, New York, Cold Spring Harbor Laboratory Press.

Schägger H, von Jagow G (1987) Tricine-Sodium Dodecyl Sulfate-Polyacrylamide Gel Electrophoresis or the Separation of Proteins in the Range from 1 to 100 kDa. Anal Biochem, 166:368-379.

Skaleric U, Allen J, Smith J, Mergenhagen S, Wahl S (1991) Inhibiton of Reactive Oxygen Intermediates Suppress Bacterial cell Well-Induced Arthritis. J Immunol, 147:2559-2564.

Strömqvist M, Holgersson J, Samuelson B (1991) Glycosylation of Extracellular Superoxide Dismutase Studied by High-Performance Liquid Chromatography and Mass Spectrometry. J Chromatography, 548:293-301.

Tibell L, Hjalmarsson K, Edlund T, Skogman G, Engstrom Å, Marklund S (1987) Expression of Human Extracellular Superoxide Dismutase in Chinese Hamster Ovary Cells and Characterization of the Product. Proc Natl Acad Sci (USA), 84:6634-6638.

Trout A, McHeyzer-Williams M, Pulendran B, Nossal G. (1992) Ligation-anchored PCR: A simple amplification technique with single-sided specificity. Proc Natl Acad Sci (USA), 89:9823-9825.

Vanfleteren J, Raymackers J, Van Bun S, Meheus L (1992) Peptide Mapping and Microsequencing of Proteins Separated by SDS-PAGE after Limited *in Situ* Acid hydrolysis. BioTechniques, 12:550-557.

Willems J, Joniau M, Cinque S, Van Damme J (1989) Human granulocyte chemotactic peptide (IL-8) as a specific neutrophil degranulator: Comparison with other monokines. Immunology 67:540-542.

Wray W, Boulikas T, Wray V, Hancock R (1981) Silver Staining of Proteins in Polyacrylamide Gels. Anal Biochem 118:197-203.

Zagursky R, Baumeister K, Lomay N, Berman M (1985) Rapid and easy sequencing of large linear double-strand DNA and supercoiled plasmid DNA. Gene Anal Tech 2:89-94.

Zhu J, Kempenaers W, Vanderstraeten D, Contreras R, Fiers W (1985) A method for fast and pure DNA elution from agarose gels by centrifugal filtration. Biotechnology 3:1014-1016.

EP 0 627 486 A1

## Claims

1. An extracellular superoxide dismutase B form (EC-SOD B) polypeptide, in substantially pure form, characterized by an amino acid sequence having:
   * at least 50% homology, preferably at least 55% homology, more preferably having at least 60% homology to the amino acid sequence spanning positions 1 to 98 as shown in SEQ ID NO 1 or 2, and/or,
   * at least 90% homology, preferably at least 92% homology, more preferably having at least 95% homology to the amino acid sequence spanning positions 99 to 198 as shown in SEQ ID NO 1 or 2, and/or,
   * at least 52% homology, preferably at least 55% homology, more preferably having at least 60% homology to the amino acid sequence spanning positions 199 to 224 as shown in SEQ ID NO 1 or 2.

2. A polypeptide according to claim 1, wherein said EC-SOD B polypeptide comprises an amino acid sequence having at least 67% homology, preferably at least 70% homology, most preferably at least 75% homology to the 224 amino acids long mature polypeptide amino acid sequence as shown in SEQ ID NO 1 or 2.

3. A polypeptide according to any of claims 1 or 2 wherein said EC-SOD B polypeptide is further characterized by its biological activity involving:
   - the dismutation of reactive oxygen intermediates produced by activated human neutrophils and
   - its binding affinity for human neutrophils.

4. A polypeptide according to any of claims 1 to 3 wherein said EC-SOD B polypeptide is further characterized as:

* having a native molecular weight of about 85 kDa ± 20 kDa as determined by gelfiltration on a Toyopearl HW 55S and on a Superose 12 collumn, and with a subunit molecular weight ranging from about 30 kDa to about 38 kDa as determined by SDS-PAGE,
* having an isoelectric point ranging from about 6.1 to about 7.3, as determined by isoelectrofocusing,
* eluting from a heparin-Sepharose column between 0.1 and 0.5 M NaCl, and preferably between 0.3 and 0.4 M NaCl,
* being secreted by mammalian cells,
* having binding affinity for human neutrophils.

5. A polypeptide according to any of claims 1 or 4 further characterized by the 224 amino acid mature peptide sequence as represented in SEQ ID NO 1 or 2.

6. A polypeptide according to any one of claims 1 to 5, in a glycosylated form.

7. A polypeptide according to any one of claims 1 to 6 liable to be obtained according to a process including at least the following steps:

- growing cells known to secrete the polypeptide to be isolated and purified in a suitable culture medium and harvesting the cells to recover the culture medium,
- performing a heparin-Sepharose chromatography with the recovered culture medium and collecting the fractions showing the SOD biological activity and/or the neutrophil binding activity and eluting between 0.1 and 0.5 M NaCl, preferably between 0.3 and 0.4 M NaCl,
- performing a hydrophobic interaction chromatography on biologically active fractions obtained after heparin-Sepharose chromatography and collecting the fractions showing the SOD biological activity and/or the neutrophil binding activity and eluting between 400 nM and 200 nM $(NH_4)_2SO_4$ in 50 mM sodiumphosphate (pH 7.0),
- performing a heparin-HPLC chromatography with the biologically active fractions obtained from the hydrophobic interaction chromatography and collecting the fractions having the SOD activity and/or the neutrophil binding activity between 250 and 300 mM NaCl, 50 mM Tris-HCl pH 7.1,
- performing a reversed-phase HPLC chromatography with the biologically active fractions obtained from the heparin HPLC chromatography, preferably C4, and isolating the fractions showing the biological activity and/or the neutrophil binding activity.

8. A polypeptide or peptide comprising a contiguous sequence of at least 10 amino acids of an extracellular B form polypeptide according to any of claims 1 to 7, with said polypeptide or peptide presenting at least one of the biological activities of the extracellular B form as defined in any of claims 3 or 4, and with said peptide or polypeptide containing in its contiguous sequence at least one amino acid different from the corresponding region of human EC-SOD C.

9. A polypeptide comprising a contiguous sequence of at least 10 amino acids comprised in the 224 amino acids long mature peptide amino acid sequence of EC-SOD B as shown in SEQ ID NO 1 or 2, more particularly a polypeptide comprising a contiguous sequence of at least one of the following regions of the EC-SOD B mature polypeptide:

- amino acids spanning position 199 to 213,
- amino acids spanning position 1 to 55,
- amino acids spanning positions 53 to 72,
- amino acids spanning positions 71 to 89,
- amino acids spanning positions 87 to 102,
- amino acids spanning positions 100 to 108,
- amino acids spanning positions 119 to 127,
- amino acids spanning positions 133 to 141,
- amino acids spanning positions 138 to 156,
- amino acids spanning positions 157 to 165,
- amino acids spanning positions 173 to 181,
- amino acids spanning positions 181 to 189,
- amino acids spanning positions 195 to 214,

- amino acids spanning positions 219 to 224.

10. A polynucleic acid in substantially isolated form comprising a contiguous sequence of at least 10 nucleotides selected from:
(a) the polynucleic acid sequences which code for any of the EC-SOD B polypeptides according to any of claims 1 to 9.
(b) the polynucleic acid sequences which hybridize to any of the polynucleic acids as defined in (a), or,
(c) the polynucleic acid sequences which are degenerate as a result of the genetic code to the polynucleic acid sequences as defined in (a) or (b) and which either code for a polypeptide according to any of claims 1 to 9, or hybridize to a polynucleic acid which codes for a polypeptide according to any of claims 1 to 9.

11. A polynucleic acid according to claim 10 comprising a contiguous sequence of at least 10 nucleotides selected from:
(a) the polynucleic acid sequence as shown in SEQ ID NO 1, or,
(b) the polynucleic acid sequences which hybridize to the polynucleic acid as shown in SEQ ID NO 1, or,
(c) the polynucleic acid sequences which are degenerate as a result of the genetic code to the polynucleic acid sequences as shown in SEQ ID NO 1, or to the polynucleic acid sequences which hybridize to the polynucleic acid sequences as shown in SEQ ID NO 1.

12. A polynucleic acid sequence according to any of claims 10 or 11 comprising a cDNA or a full length genomic clone encoding any of the polypeptides according to claims 1 to 7, with said clone being obtained by a process comprising essentially the following steps:
- preparing RNA from mammalian cells (step 1),
- performing an amplification reaction of mRNA with primers essentially consisting of, or comprising, nucleotide sequences containing at least part of the nucleotide sequence encoding a polypeptide according to any of claim 1 or 8, to obtain amplified products (step 2),
- screening of a mammalian cDNA library to obtain full length cDNA clones by using the amplified products obtained in step 2, as a probe for hybridization (step 3),
- screening of a genomic library to obtain the full length genomic clone by using the above-mentioned amplified products of step 2 or cDNA clones obtained in step 3 as a probe for hybridization (step 4).

13. A polynucleic acid sequence comprising at least 10 nucleotides according to any of claims 10 to 12, for use as a specific hybridization probe for detecting the presence of a polynucleic acid encoding any of the polypeptides according to claims 1 to 8, or the complement thereof.

14. A polynucleic acid sequence according to any of claims 10 to 12, for use as a primer for amplification of a polynucleic acid encoding any of the polypeptides according to any one of claims 1 to 9, or the complement thereof.

15. A recombinant vector, particularly for cloning and/or expression, with said recombinant vector comprising a vector sequence, itself comprising a coding sequence which comprises a DNA polynucleotide according to any of claims 10 to 12, and wherein the coding sequence is operably linked to a control sequence capable of providing for the expression of the coding sequence by the specific host cell.

16. A host cell transformed by a recombinant vector according to claim 15.

17. A recombinant polypeptide produced by transforming a recombinant vector according to claim 15 into a suitable prokaryotic or eukaryotic host according to claim 16, and culturing said transformed cellular host under conditions enabling the expression of said insert.

18. A method of producing a recombinant polypeptide according to claim 17 comprising incubating a host cell according to claim 16 under conditions that provide for the expression of the coding sequence.

19. An antibody, particularly a monoclonal antibody, characterized in that it is specifically directed against an antigenic determinant of a polypeptide according to any of claims 1 to 9.

20. Anti-idiotype antibody raised against the antibody according to claim 19.

21. Antisense peptides derived from the polypeptides according to any of claims 1 to 9 or derived from the polynucleic acid sequences according to any of claims 10 to 12.

22. A polypeptide according to any of claims 1 to 9, for providing superoxide dismutase therapy in any illness state related to the production of superoxide radicals, more particularly in any illness state where binding to neutrophils is highly advantageous.

23. Pharmaceutical compositions, containing as active subtance, anyone of the polypeptides according to any of claims 1 to 9, or of the anti-idiotype antibodies according to claim 20 in association with a pharmaceutically acceptable vehicle.

24. Use of a polypeptide according to any of claims 1 to 9, or of an anti-idiotype antibody according to claim 20, as active substance for the preparation of a drug useful in the treatment of all types of lung tissue damage, such as hyperoxia, respiratory distress syndrome, emfysema, or useful in the treatment of radiation damage, burns, ulcers, retinopathy in premature infants, ischemic reperfusion damage, cerebrovascular damage, organ transplantation atherosclerosis, patients carrying glutathion synthase deficient neutrophils, all types of symptoms associated with aging, such as cataract, or for the cold storage of organs.

25. Process for purification of a polypeptide according to any of claims 1 to 9, comprising an affinity chromatography using immobilized antisense peptides according to claim 21, or antibodies according to claim 19.

Figure 1

Figure 2

Figure 3

Figure 4

Figure 5

Figure 6

```
Human rhEC-SOD C   - WTGE---DSAEPNSDSAEWIRDMYAKVTEIWQEVMQRRDD -37
                     : ::        :  :.      :  .:  :   :    .:  ::.   .
Rat EC-SOD B       - MSDTGESGVDLADRL-DLVEKIGDTHSKDLEIXMXLXKQXXA -41
                     ◄————————————————— N-term ——————————————►


Human rhEC-SOD C - DGT-LHAACQVQPSATLDAAQPRVTGVVLFRQLAPRAKLDAFFALEGFPT -86
                         :         ::  .:.:.::::::  :  ...:.: : ::::.
Rat EC-SOD B     -                 LPPDQPQITGLVLFRQLGPSSRLEASFNLEGFPA
                                   ◄———————————————— 13 kD CNBr ————
                                   ◄————— 20.8 kD ——————►


Human rhEC-SOD C - EPNSSSRAIHVHQFGDLSQGCESTGPHYNPLAVPHPQHPGDFGNFAVRDG -136
                     :  :  :   ::::        :  ::  :::::  :  ::  ::  ::  :::::::  ::
Rat EC-SOD B     - EQNXSXXAIH      LXQGXESTGPXYXPLGVPXPQXPGDFGNFVVR
                   ——————————————►      ◄——————— AH 16 ——————►◄—AH23—►


Human rhEC-SOD C - SLWRYRAGLAASLAGPHSIVGRAVVVHAGEDDLGRGGNQASVENGNAGRR -186
                     ::.::::::::.:::::::::::::: : :::::  :::::
Rat EC-SOD B     -           GLATSLAGPHSILGRAVVVHAGEDDLGXGXNQASVXNGNAGXX
                             ◄——————— 10 kD ——————►               ◄—H2—►
                                                          ◄————— AH 22 —————
```

EP 0 627 486 A1

Human rhEC-SOD C - LACCVVGVCGPGLWERQAREHSERKKKRRRESECKAA -222

Rat EC-SOD B - LAXXVVGTSNSEAXESQTK---ERKKRRRESECKTT

T 8

H1

Figure 6 (continued)

Figure 7

```
        10         20         30         40         50         60
         |          |          |          |          |          |
GGCTCACAAGCAGCTGGCCAGTTCTGGGGAGGCAGCTCAGAGGCTCTTTCTCAGGCCTCT

        70         80         90        100        110        120
         |          |          |          |          |          |
AGCTGGGTCTGTCCTGTACTTCACCAGAGGAAAAACGTTCTTGGGAGAGCTTGTCAGGTG

       130        140        150        160        170        180
         |          |          |          |          |          |
TGGAACCTCAGCCATGGTGGCCTTCTTGTTCTGCAACCTGCTACTGGTGGCCTGTGGCTC
                   M   V   A   F   L   F   C   N   L   L   L   V   A   C   G   S

       190        200        210        220        230        240
         |          |          |          |          |          |
TGTCACCTGGACCATGTCAGATACCGGAGAGTCCGGTGTCGACTTAGCAGACCGGCTTGA
  V   T   W   T   M   S   D   T   G   E   S   G   V   D   L   A   D   R   L   D

       250        260        270        280        290        300
         |          |          |          |          |          |
CCTGGTTGAGAAGATAGGCGACACGCACTCCAAAGACCTGGAGATCTGGATGGAGCTAGG
  L   V   E   K   I   G   D   T   H   S   K   D   L   E   I   W   M   E   L   G

       310        320        330        340        350        360
         |          |          |          |          |          |
AAAACAACGGGAGGCGGATGCCAGGGAGATGCACGCAGTCTGCAGGGTACAGCCCTCAGC
  K   Q   R   E   A   D   A   R   E   M   H   A   V   C   R   V   Q   P   S   A
```

EP 0 627 486 A1

```
           370          380          390          400          410          420
            |            |            |            |            |            |
CATGCTGCCTCCCGATCAGCCACAGATCACAGGCTTGGTCCTCTTCCGGCAGCTGGGGCC
  M   L   P   P   D   Q   P   Q   I   T   G   L   V   L   F   R   Q   L   G   P

           430          440          450          460          470          480
            |            |            |            |            |            |
CAGCTCCAGACTTGAGGCCTCCTTCAATCTGGAGGGCTTCCCAGCCGAGCAGAACACCTC
  S   S   R   L   E   A   S   F   N   L   E   G   F   P   A   E   Q   N   T   S

           490          500          510          520          530          540
            |            |            |            |            |            |
CAACCACGCCATCCACGTGCATGAGTTCGGGGACCTGAGCCAGGGCTGCGAGTCCACCGG
  N   H   A   I   H   V   H   E   F   G   D   L   S   Q   G   C   E   S   T   G

           550          560          570          580          590          600
            |            |            |            |            |            |
ACCACACTACAACCCGCTGGGTGTGCCGCACCCACAGCACCCGGGGGACTTCGGCAACTT
  P   H   Y   N   P   L   G   V   P   H   P   Q   H   P   G   D   F   G   N   F

           610          620          630          640          650          660
            |            |            |            |            |            |
CGTGGTGCGCGATGGCCGCCTTTGGAAGCATCGAATGGGCCTGGCCACGTCACTGGCCGG
  V   V   R   D   G   R   L   W   K   H   R   M   G   L   A   T   S   L   A   G
```

Figure 7 (continued 1)

EP 0 627 486 A1

```
           670          680          690          700          710          720
            |            |            |            |            |            |
ACCGCACTCGATCTTGGGCCGCGCTGTGGTGGTCCACGCTGGCGAGGACGACCTGGGTAA
  P   H   S   I   L   G   R   A   V   V   V   H   A   G   E   D   D   L   G   K

           730          740          750          760          770          780
            |            |            |            |            |            |
AGGTGGCAACCAGGCCAGCGTGCAGAACGGCAACGCAGGTCGCCGGCTCGCCTGCTGCGT
  G   G   N   Q   A   S   V   Q   N   G   N   A   G   R   R   L   A   C   C   V

           790          800          810          820          830          840
            |            |            |            |            |            |
GGTAGGCACCAGCAACTCGGAGGCCTGGGAGAGCCAGACAAAGGAGCGCAAGAAGCGGCG
  V   G   T   S   N   S   E   A   W   E   S   Q   T   K   E   R   K   K   R   R

           850          860          870          880          890          900
            |            |            |            |            |            |
GCGGGAGAGCGAGTGCAAGACCACTTAAGCATCACCCAGGGCCGCCTAGCCTAGCTGCTG
  R   E   S   E   C   K   T   T

           910          920          930          940          950          960
            |            |            |            |            |            |
CGCGCATAGATGCCTCCACACGCGCCCTCTAGACGCCTCCAGTCATCCTAGAGGTCTCTG

           970          980          990         1000         1010         1020
            |            |            |            |            |            |
GGTGTCCTAGACTGACGCTTCCCAGACACCTCAATCGCCTCTGTGCGCCCCACACTCTTC
```

Figure 7 (continued 2)

EP 0 627 486 A1

```
           1030        1040        1050        1060        1070        1080
             |           |           |           |           |           |
CACATACCCCAGACACCTCTGTATGGCTCAGATGCCTTCAAGAACCTCCTCGGCCACGTC

           1090        1100        1110        1120        1130        1140
             |           |           |           |           |           |
CACGAGCCCCAGATGTTCCCACGTGCCCTGGGCACTGTTCTCGGAGACCAGGACACTTTT

           1150        1160        1170        1180        1190        1200
             |           |           |           |           |           |
TTGTAACCTAGGAATCCTTCACACCTATGCACTCCACAGACCAACTCCTTCGTGCTCTAG

           1210        1220        1230        1240        1250        1260
             |           |           |           |           |           |
GTCCACCTCGAACTACTTTATGCCCCAAGACAATCCCATAAGCCCCTAGCATCCCCTTTG

           1270        1280        1290        1300        1310        1320
             |           |           |           |           |           |
AAACAGTCTTTGAGTTTGCTCCCAGAGAATTCCCCGCTTACCCCCAGAGGTCGAATGTGC

           1330        1340        1350        1360        1370        1380
             |           |           |           |           |           |
GCAGATAACTCTCCTTTTACTCTGAGGACATCCCAGTGGACCTTCTAGAGAACTCCCTTG

           1390        1400        1410        1420        1430        1440
             |           |           |           |           |           |
GGGTGTTCTGAAATATCACCACCCCACTTCCTTCTGCCCCCTTTTGTTTTCTTTCTGTCC
```

Figure 7 (continued 3)

EP 0 627 486 A1

```
      1450          1460          1470          1480          1490          1500
        |             |             |             |             |             |
CCTAGCACCCGAGACTTCTCTCTTCCCTAGAGACCTCGTTTGTCTTCCCCTTGTTCCTCC

      1510          1520          1530          1540          1550          1560
        |             |             |             |             |             |
TAGGGCTCTGGGACCACCCTGACACACACACACACACACACACACACACACACACACACA

      1570          1580          1590          1600          1610          1620
        |             |             |             |             |             |
CACACACACACACATCCCTAAGATTCCATGTTCCTGATCACCTCCTGCCGGGCCCCTGGT

      1630          1640          1650          1660          1670          1680
        |             |             |             |             |             |
TCTGTTTTCATCTGTTTCCCATATGGTGCCTGCACCCCAAGGAGAGCAGCTCCTCCGAGA

      1690          1700          1710          1720
        |             |             |             |
GTATTTGACAACCTTTATGCTGCTCATTAAAACCACAGCAATTCAAAAA
```

Figure 7 (continued 4)

```
HUECSODC   -    WTGE---DSAEPNSDSAEWIRDMYAKVTEIWQE-VMQRRDD -37
                :::     : :      : .: : :   .:  ::: ::  ::   :
RAECSODB   - MSDTGESGVDLA-DRLDLVEKIGDTHSKDLEIWMELGKQREAD -41


HUECSODC   - DGTLHAACQVQPSATLDAAQPRVTGVVLFRQLAPRAKLDAFFALEGFPT -86
                :::.: :::::: :     :: .::.::::::.: ...:.: : :::::.
RAECSODB   - AREMHAVCRVQPSAMLPPDQPQITGLVLFRQLGPSSRLEASFNLEGFPA -91


HUECSODC   - EPNSSSRAIHVHQFGDLSQGCESTGPHYNPLAVPHPQHPGDFGNFAVRDG -136
                : : :    ::::: :::::::::::::::::::::.::::::::::::.:::
RAECSODB   - EQNTSNHAIHVHEFGDLSQGCESTGPHYNPLGVPHPQHPGDFGNFVVRDG -141


HUECSODC   - SLWRYRAGLAASLAGPHSIVGRAVVVHAGEDDLGRGGNQASVENGNAGRR -186
                ::. : :::.:::::::.::::::::::::::.::::::: :::::::
RAECSODB   - RLWKHRMGLATSLAGPHSILGRAVVVHAGEDDLGKGGNQASVQNGNAGRR -191


HUECSODC   - LACCVVGVCGPGLWERQAREHSERKKRRRESECKAA -222
                ::::::::      :: :       :::::::::::..
RAECSODB   - LACCVVGTSNSEAWESQ---TKERKKRRRESECKTT -224
```

Figure 8

EP 0 627 486 A1

RAECSODB  -  GGCTCACAAGCAGCTGGCCCAGTTCTGGGGAGGCAGCTCAGAGGCTCTTTC  -50

HUECSODC  -  CTGGGT--GCAGCTCT-----CTTTT  -19

RAECSODB  -  TCAGGCCCTCTAGCTGGGTCTGTCCTGTACTTCACCAGAGGAAAAACGTTC  -100

HUECSODC  -  -CAGGA----GAGAAAGCTCTC  -36

Start signal

RAECSODB  -  TTGGGAGAGCTTGTCAGGTGTGGAACCTCAGCCATGGTGGCCTTCTTGTT  -150

HUECSODC  -  TTGGGAGGAGCTGGAAAGGTGCCCGACTCCAGCCATGCTGGCGCTACTGTG  -86

Start protein

RAECSODB  -  CTGCAACCTGCTACTGGTGGCCTGTCTCTGTCACCTGGACCATGTCAG  -200

HUECSODC  -  TTCCTGCCTGCTCCTGGCAGCCGGGGTGCCCTCGGACGG-----  -130

RAECSODB  -  ATACCGGAGAGTCCGGTGTCGACTTAGCAGA--CCGGCT-TGACCTGGTT  -247

HUECSODC  -  ------GCGAGGACTCGGCGGGAGCCCAACTCTGACTCGGGCG  -165

RAECSODB  -  GAGAAGATAGGCGACACGCACTCCAAAGACCTGGAGATCTGGATGGAGCT  -297

HUECSODC  -  GAGTGGGATCCGAGACATGTACGCCAAGGTCACGGAGAGATCTGGCAGGAGGT  -215

Figure 9

```
RAECSODB  - AGGAAAACAACGGGAGGCGGATGCCAGGGAGATGCACGCAGTCTGCAGGG -347
            | |   ||||| |   || |  || |      | |||||| | |||| ||
HUECSODC  - CATGCAGCGGCGGGACGACGACGGCACG---CTCCACGCCGCCTGCCAGG -262

RAECSODB  - TACAGCCCTCAGCCATGCTGCCTCCCGATCAGCCACAGATCACAGGCTTG -397
            | ||||| || |||| ||||     ||| ||||| | | | || ||| |
HUECSODC  - TGCAGCCGTCGGCCACGCTGGACGCCGCGCAGCCCCGGGTGACCGGCGTC -312

RAECSODB  - GTCCTCTTCCGGCAGCTGGGGCCCAGCTCCAGACTTGAGGCCTCCTTCAA -447
            |||||||||||||||| |||||  |  | |||| || || ||| ||| ||||
HUECSODC  - GTCCTCTTCCGGCAGCTTGCGCCCCGCGCCAAGCTCGACGCCTTCTTCGC -362

RAECSODB  - TCTGGAGGGCTTCCCAGCCGAGCAGAACACCTCCAACCACGCCATCCACG -497
             |||||||||||||||| |||||| ||||| ||||| || |||||||||||
HUECSODC  - CCTGGAGGGCTTCCCGACCGAGCCGAACAGCTCCAGCCGCGCCATCCACG -412

RAECSODB  - TGCATGAGTTCGGGGACCTGAGCCAGGGCTGCGAGTCCACCGGACCACAC -547
            ||||    |||||||||||||||||||||||||||||||||||||  |||
HUECSODC  - TGCACCAGTTCGGGGACCTGAGCCAGGGCTGCGAGTCCACCGGGCCCCAC -462

RAECSODB  - TACAACCCGCTGGGTGTGCCGCACCCACAGCACCCGGGGGACTTCGGCAA -597
            |||||||||||||  ||||||||||||| |||||||||| ||||||||||
HUECSODC  - TACAACCCGCTGGCCGTGCCGCACCCGCAGCACCCGGGCGACTTCGGCAA -512

RAECSODB  - CTTCGTGGTGCGCGATGGCCGCCTTTGGAAGCATCGAATGGGCCTGGCCA -647
            ||||||  ||| ||||| |||  |||| |||| | |  || |||||||||
HUECSODC  - CTTCGCGGTCCGCGACGGCAGCCTCTGGAGGTACCGCGCCGGCCTGGCCG -562
```

Figure 9 (continued 1)

EP 0 627 486 A1

EP 0 627 486 A1

```
RAECSODB   -  CGTCACTGGCCGGACCGCACTCGATCTTGGGCCGCGCTGTGGTGGTCCAC   -697
              |  |  | ||   | |   | |  ||||||||| |||  |||||||| ||  ||||| | ||||||||
HUECSODC   -  CCTCGCTCGCGGGCCCGCACTCCATCGTGGGCCGGGCCGTGGTCGTCCAC   -612

RAECSODB   -  GCTGGCGAGGACGACCTGGGTAAAGGTGGCAACCAGGCCAGCGTGCAGAA   -747
              ||||||||||||||||||||||||||||     || ||||||||||||||||||||||||| ||||
HUECSODC   -  GCTGGCGAGGACGACCTGGGCCGCGGCGGCAACCAGGCCAGCGTGGAGAA   -662

RAECSODB   -  CGGCAACGCAGGTCGCCGGCTCGCCTGCTGCGTGGTAGGCACCAGCAACT   -797
              |||  ||||||  ||   || | |||||  ||||||||||||||||||  |||        ||
HUECSODC   -  CGGGAACGCGGGCCGGCGGCTGGCCTGCTGCGTGGTGGGCGTGTGCGGGC   -712

RAECSODB   -  CGGAGGCCTGGGAGAGCCAGACAAAGGAGC----------GCAAGAAGCGG   -838
              |  |  |    ||||||||  |||||| |     |||||             ||||||||||||
HUECSODC   -  CCGGGCTCTGGGAGCGCCAGGCGCGGGAGCACTCAGAGCGCAAGAAGCGG   -762
```

Stop protein

```
RAECSODB   -  CGGCGGGAGAGCGAGTGCAAGACCACTTAAGCATCACCCAGGGCCGCCTA   -888
              |||||  ||||||||||||||||||||  ||   |   |   |||
HUECSODC   -  CGGCGCGAGAGCGAGTGCAAGGCCGCCTGAGCG-----------------   -795

RAECSODB   -  GCCTAGCTGCTGCGCGCATAGATGCCTCCACACGCGCCCTCTAGACGCCT   -938
                                   | |              || |||| ||                  ||
HUECSODC   -  --------------CGG-------CCCCCACCCGG---------CGGCGG   -815

RAECSODB   -  CCAGTCATCCTAGAGGTCTCTGGGTGTCCTAGACTGACGCTTCCCAGACA   -988
              ||||   |  ||   ||||  |    ||  |  |   |||||  |
HUECSODC   -  CCAGGGACCCCCGAGGCCCCCCTCTGCCTTTGAG-----CTTCTCC----   -856
```

Figure 9 (continued 2)

```
RAECSODB - CCTCAATCGCCTCTGTGCGCCCCACACTCTTCCACATACCCCAGACACCT -1038
                ||||                   |  |  |||  || ||      |||
HUECSODC - ----------TCTGC--------------TCCAACAGACACCTTCCACT- -881

RAECSODB - CTGTATGGCTCAGATGCCTTCAAGAACCTCCTCGGCC-ACGTCCACGAGC -1087
           |||     |  ||||       ||||         ||| ||    |  |||
HUECSODC - CTGA-GGTCTCAC----CTTCGC-------CTCTGCTGAAGTCT------ -913

RAECSODB - CCCAGATGTTCCCACGTGCCCTGGGCACTGTTCTCGGAGACCAGGACACT -1137
                   |||      |||||      |||                 |||
HUECSODC - ----------CCCCGCAGCCCTCTCCACC-------------CAGA----- -936

RAECSODB - TTTTTGTAACCTAGGAATCCTTCACACCTATGCACTCCACAGACCAACTC -1187
                   ||    |||| |    |||  |    |  |  |||         |
HUECSODC - --------------GGTCTCCCTA-TACCGAGACCCACCAT---------C -963

RAECSODB - CTTCGTGCTCTAGGTCCACCTCGAACTACTTTATGCCCCAAGACAATCCC -1237
           ||||     |    ||| ||                   ||||||
HUECSODC - CTTCCATCCTGAGGACCG-------------------CCCCAAC-------- -988

RAECSODB - ATAAGCCCCTAGCATCCCCTTTGAAACAGTCTTTGAGTTTGCTCCCAGAG -1287
                 |||  |  |  ||||        || | |   |  || ||
HUECSODC - -------CCTCGGAGCCCCC------CACTCAGTAGGTCTGA-------- -1017

RAECSODB - AATTCCCCGCTTACCCCCAGAGGTCGAATGTGCGCAGATAACTCTCCTTT -1337
            |  ||| |  ||               ||| |  |   || |  ||  |
HUECSODC - -AGGCCTCCATTT----------------GTACCGAA---ACACCCCGCT -1047
```

Figure 9 (continued 3)

EP 0 627 486 A1

EP 0 627 486 A1

```
RAECSODB  - TACTCTGAGGACATCCCAGTGGACCTTCTAGAGAACTCCCTTGGGGTGTT  -1387
            ||  ||||    |  |||  ||                 |||||    ||    |
HUECSODC  - CACGCTGACAGCCTCCTAGGC---------------TCCCTGAGGTACCT  -1082

RAECSODB  - CTGAAATATCACCACCCCACTTCCTTCTGCCCCCTTTTGTTTTCTTTCTG  -1437
            |               |||||        |||  ||  ||||
HUECSODC  - TTC---------CACCCA-GACCCTCCTTCCCACC---------------  -1108

RAECSODB  - TCCCCTAGCACCCGAGACTTCTCTCTTCCCTAGAGACCTCGTTTGTCTTC  -1487
             ||  ||    ||  ||||||| |      |||        ||||    |||||
HUECSODC  - --CCATAAGCCCTGAGACTCCCGCCTTTG------ACCTG-ACGATCTTC  -1149

RAECSODB  - CCCTT------------GTTCCTCCTAGG-GCTCTGGGACCACCCTGACA  -1524
            ||||  |           |||||||||||||  ||||  |  ||  |  |  |||
HUECSODC  - CCCCTTCCCGCCTTCAGGTTCCTCCTAGGCGCTCAGAGGCCGCTCTGGGG  -1199

RAECSODB  - CACACACACACACACACACACACACACACACACACACACACACACACACA  -1574
               |  |     |  |  |||  |     |  |||  |||            ||
HUECSODC  - GGTTGCCTCGAGTCCCCCCACCCCTCCCCACCCACC--------ACCGC  -1240

RAECSODB  - TCCCTAAGATTCCATGTTCCTGATCACCTCCTGCCGGGCCCCTGGTTCTG  -1624
            ||||    |     |  |     |  ||  |     |||  ||||     |    | |
HUECSODC  - TCCCGCGGCAAGCCAGC--CCGTGCAACGGAAGCCAGGCCAACTGCCCCG  -1288

RAECSODB  - --TTTTCATCTGTTTCCCATATGGTGCCTGCACCCCAAGGAGAGCAGCTC  -1672
            |  ||||| |||||||| |||         ||   |||||||  ||||||| ||||
HUECSODC  - CGTCTTCAGCTGTTTCGCATCCA---CCGCCACCCCACTGAGAGCTGCTC  -1335
```

Figure 9 (continued 4)

```
RAECSODB   -  CTCCGAGAGTAT--TTGACAACCTTTATGCTGCTCATTAAAACCACAGCA -1720
              ||   |  |  |  |  ||   |||  |||||||||  ||  |  |   ||||||||   ||||||
HUECSODC   -  CTTTGGGGGAATGTTTGGCAACCTTTGTGTTACAGATTAAAAATTCAGCA -1385

RAECSODB   -  ATTCAAAAA -1729
              ||||
HUECSODC   -  ATTC         -1389
```

Figure 9 (continued 5)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 492 447 (TOYO JOZO CO., LTD.) 1 July 1992 <br> * page 8 - page 9, line 35 * <br> --- | 9-18 | C12N9/02 <br> C12N15/53 <br> C12P21/08 <br> A61K37/48 |
| X | WO-A-87 01387 (SYN-TEK AB) 12 March 1987 <br> * page 6 - page 7 * <br> --- | 9-19 | |
| X | WO-A-91 04315 (SYMBICOM AKTIEBOLAG) 4 April 1991 <br> * example 4 * <br> ----- | 8 | |

TECHNICAL FIELDS
SEARCHED      (Int.Cl.5)

C12N
A61K

The present search report has been drawn up for all claims

| Place of search | Date of completion of the search | Examiner |
|---|---|---|
| THE HAGUE | 27 October 1993 | CUPIDO, M |

EPO FORM 1503 03.82 (P04C01)

SEQUENCE LISTING

(1) GENERAL INFORMATION:

    (i) APPLICANT:
       (A) NAME: INNOGENETICS N.V
       (B) STREET: Industriepark Zwijnaarde 7 box 4
       (C) CITY: Ghent
       (E) COUNTRY: BELGIUM
       (F) POSTAL CODE (ZIP): B-9052
       (G) TELEPHONE: 00 32 91 410 711
       (H) TELEFAX: 00 32 91 410 799

    (ii) TITLE OF INVENTION: New extracellular superoxide dismutase, a
       process for preparing the same and compositions containing
       the same

    (iii) NUMBER OF SEQUENCES: 19

    (iv) COMPUTER READABLE FORM:
       (A) MEDIUM TYPE: Floppy disk
       (B) COMPUTER: IBM PC compatible
       (C) OPERATING SYSTEM: PC-DOS/MS-DOS
       (D) SOFTWARE: PatentIn Release #1.0, Version #1.25 (EPO)

(2) INFORMATION FOR SEQ ID NO: 1:

    (i) SEQUENCE CHARACTERISTICS:
       (A) LENGTH: 1729 base pairs
       (B) TYPE: nucleic acid
       (C) STRANDEDNESS: single
       (D) TOPOLOGY: linear

    (ii) MOLECULE TYPE: cDNA

    (vi) ORIGINAL SOURCE:
       (A) ORGANISM: Rat
       (F) TISSUE TYPE: glioma

    (vii) IMMEDIATE SOURCE:
       (A) LIBRARY: Rat C6 glioma lambda gt11 cDNA library

    (ix) FEATURE:
       (A) NAME/KEY: CDS
       (B) LOCATION: 134..865

    (ix) FEATURE:
       (A) NAME/KEY: sig_peptide
       (B) LOCATION: 134..193

    (ix) FEATURE:
       (A) NAME/KEY: mat_peptide
       (B) LOCATION: 194..865

    (ix) FEATURE:
       (A) NAME/KEY: 5'UTR
       (B) LOCATION: 1..133

    (ix) FEATURE:
       (A) NAME/KEY: 3'UTR
       (B) LOCATION: 866..1729

    (xi) SEQUENCE DESCRIPTION: SEQ ID NO: 1:

23

GGCTCACAAG CAGCTGGCCA GTTCTGGGGA GGCAGCTCAG AGGCTCTTTC TCAGGCCTCT 60

AGCTGGGTCT GTCCTGTACT TCACCAGAGG AAAAACGTTC TTGGGAGAGC TTGTCAGGTG 120

```
TGGAACCTCA GCC ATG GTG GCC TTC TTG TTC TGC AAC CTG CTA CTG GTG       169
            Met Val Ala Phe Leu Phe Cys Asn Leu Leu Leu Val
            -20                 -15                 -10

GCC TGT GGC TCT GTC ACC TGG ACC ATG TCA GAT ACC GGA GAG TCC GGT       217
Ala Cys Gly Ser Val Thr Trp Thr Met Ser Asp Thr Gly Glu Ser Gly
            -5                  1                   5

GTC GAC TTA GCA GAC CGG CTT GAC CTG GTT GAG AAG ATA GGC GAC ACG       265
Val Asp Leu Ala Asp Arg Leu Asp Leu Val Glu Lys Ile Gly Asp Thr
        10              15              20

CAC TCC AAA GAC CTG GAG ATC TGG ATG GAG CTA GGA AAA CAA CGG GAG       313
His Ser Lys Asp Leu Glu Ile Trp Met Glu Leu Gly Lys Gln Arg Glu
25                  30              35                  40

GCG GAT GCC AGG GAG ATG CAC GCA GTC TGC AGG GTA CAG CCC TCA GCC       361
Ala Asp Ala Arg Glu Met His Ala Val Cys Arg Val Gln Pro Ser Ala
                45              50                  55

ATG CTG CCT CCC GAT CAG CCA CAG ATC ACA GGC TTG GTC CTC TTC CGG       409
Met Leu Pro Pro Asp Gln Pro Gln Ile Thr Gly Leu Val Leu Phe Arg
            60              65                  70

CAG CTG GGG CCC AGC TCC AGA CTT GAG GCC TCC TTC AAT CTG GAG GGC       457
Gln Leu Gly Pro Ser Ser Arg Leu Glu Ala Ser Phe Asn Leu Glu Gly
            75                  80                  85

TTC CCA GCC GAG CAG AAC ACC TCC AAC CAC GCC ATC CAC GTG CAT GAG       505
Phe Pro Ala Glu Gln Asn Thr Ser Asn His Ala Ile His Val His Glu
            90                  95                  100

TTC GGG GAC CTG AGC CAG GGC TGC GAG TCC ACC GGA CCA CAC TAC AAC       553
Phe Gly Asp Leu Ser Gln Gly Cys Glu Ser Thr Gly Pro His Tyr Asn
105                 110                 115                 120

CCG CTG GGT GTG CCG CAC CCA CAG CAC CCG GGG GAC TTC GGC AAC TTC       601
Pro Leu Gly Val Pro His Pro Gln His Pro Gly Asp Phe Gly Asn Phe
            125                 130                 135

GTG GTG CGC GAT GGC CGC CTT TGG AAG CAT CGA ATG GGC CTG GCC ACG       649
Val Val Arg Asp Gly Arg Leu Trp Lys His Arg Met Gly Leu Ala Thr
            140                 145                 150

TCA CTG GCC GGA CCG CAC TCG ATC TTG GGC CGC GCT GTG GTG GTC CAC       697
Ser Leu Ala Gly Pro His Ser Ile Leu Gly Arg Ala Val Val Val His
            155                 160                 165

GCT GGC GAG GAC GAC CTG GGT AAA GGT GGC AAC CAG GCC AGC GTG CAG       745
Ala Gly Glu Asp Asp Leu Gly Lys Gly Gly Asn Gln Ala Ser Val Gln
            170                 175                 180

AAC GGC AAC GCA GGT CGC CGG CTC GCC TGC TGC GTG GTA GGC ACC AGC       793
Asn Gly Asn Ala Gly Arg Arg Leu Ala Cys Cys Val Val Gly Thr Ser
185                 190                 195                 200

AAC TCG GAG GCC TGG GAG AGC CAG ACA AAG GAG CGC AAG AAG CGG CGG       841
Asn Ser Glu Ala Trp Glu Ser Gln Thr Lys Glu Arg Lys Lys Arg Arg
            205                 210                 215
```

```
CGG GAG AGC GAG TGC AAG ACC ACT TAAGCATCAC CCAGGGCCGC CTAGCCTAGC 895
Arg Glu Ser Glu Cys Lys Thr Thr
              220

TGCTGCGCGC ATAGATGCCT CCACACGCGC CCTCTAGACG CCTCCAGTCA TCCTAGAGGT 955

CTCTGGGTGT CCTAGACTGA CGCTTCCCAG ACACCTCAAT CGCCTCTGTG CGCCCCACAC 1015

TCTTCCACAT ACCCCAGACA CCTCTGTATG GCTCAGATGC CTTCAAGAAC CTCCTCGGCC 1075

ACGTCCACGA GCCCCAGATG TTCCCACGTG CCCTGGGCAC TGTTCTCGGA GACCAGGACA 1135

CTTTTTTGTA ACCTAGGAAT CCTTCACACC TATGCACTCC ACAGACCAAC TCCTTCGTGC 1195

TCTAGGTCCA CCTCGAACTA CTTTATGCCC CAAGACAATC CCATAAGCCC CTAGCATCCC 1255

CTTTGAAACA GTCTTTGAGT TTGCTCCCAG AGAATTCCCC GCTTACCCCC AGAGGTCGAA 1315

TGTGCGCAGA TAACTCTCCT TTTACTCTGA GGACATCCCA GTGGACCTTC TAGAGAACTC 1375

CCTTGGGGTG TTCTGAAATA TCACCACCCC ACTTCCTTCT GCCCCCTTTT GTTTTCTTTC 1435

TGTCCCCTAG CACCCGAGAC TTCTCTCTTC CCTAGAGACC TCGTTTGTCT TCCCCTTGTT 1495

CCTCCTAGGG CTCTGGGACC ACCCTGACAC ACACACACAC ACACACACAC ACACACACAC 1555

ACACACACAC ACACACACAT CCCTAAGATT CCATGTTCCT GATCACCTCC TGCCGGGCCC 1615

CTGGTTCTGT TTTCATCTGT TTCCCATATG GTGCCTGCAC CCCAAGGAGA GCAGCTCCTC 1675

CGAGAGTATT TGACAACCTT TATGCTGCTC ATTAAAACCA CAGCAATTCA AAAA         1729
```

(2)  INFORMATION FOR SEQ ID NO: 2:

        (i)  SEQUENCE CHARACTERISTICS:
            (A)  LENGTH: 244 amino acids
            (B)  TYPE: amino acid
            (D)  TOPOLOGY: linear

        (ii)  MOLECULE TYPE: protein

        (xi)  SEQUENCE DESCRIPTION: SEQ ID NO: 2:

```
Met Val Ala Phe Leu Phe Cys Asn Leu Leu Leu Val Ala Cys Gly Ser
-20              -15              -10                          -5

Val Thr Trp Thr Met Ser Asp Thr Gly Glu Ser Gly Val Asp Leu Ala
                1                5                      10

Asp Arg Leu Asp Leu Val Glu Lys Ile Gly Asp Thr His Ser Lys Asp
        15              20              25

Leu Glu Ile Trp Met Glu Leu Gly Lys Gln Arg Glu Ala Asp Ala Arg
    30              35              40

Glu Met His Ala Val Cys Arg Val Gln Pro Ser Ala Met Leu Pro Pro
  45              50              55                          60

Asp Gln Pro Gln Ile Thr Gly Leu Val Leu Phe Arg Gln Leu Gly Pro
              65              70                      75

Ser Ser Arg Leu Glu Ala Ser Phe Asn Leu Glu Gly Phe Pro Ala Glu
          80              85                      90

Gln Asn Thr Ser Asn His Ala Ile His Val His Glu Phe Gly Asp Leu
          95              100             105
```

```
Ser Gln Gly Cys Glu Ser Thr Gly Pro His Tyr Asn Pro Leu Gly Val
    110                 115                 120

Pro His Pro Gln His Pro Gly Asp Phe Gly Asn Phe Val Val Arg Asp
125                     130                 135                 140

Gly Arg Leu Trp Lys His Arg Met Gly Leu Ala Thr Ser Leu Ala Gly
            145                 150                 155

Pro His Ser Ile Leu Gly Arg Ala Val Val Val His Ala Gly Glu Asp
            160                 165                 170

Asp Leu Gly Lys Gly Gly Asn Gln Ala Ser Val Gln Asn Gly Asn Ala
        175                 180                 185

Gly Arg Arg Leu Ala Cys Cys Val Val Gly Thr Ser Asn Ser Glu Ala
    190                 195                 200

Trp Glu Ser Gln Thr Lys Glu Arg Lys Lys Arg Arg Arg Glu Ser Glu
205                 210                 215                 220

Cys Lys Thr Thr
```

(2) INFORMATION FOR SEQ ID NO: 3:

    (i) SEQUENCE CHARACTERISTICS:
        (A) LENGTH: 18 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: YES

    (iii) ANTI-SENSE: YES

    (ix) FEATURE:
        (A) NAME/KEY: misc_feature
        (B) LOCATION: 1..18
        (D) OTHER INFORMATION: /label= primer

    (ix) FEATURE:
        (A) NAME/KEY: misc_feature
        (B) LOCATION: 1
        (D) OTHER INFORMATION: N is Inosine

    (ix) FEATURE:
        (A) NAME/KEY: misc_feature
        (B) LOCATION: 10
        (D) OTHER INFORMATION: N is Inosine

    (xi) SEQUENCE DESCRIPTION: SEQ ID NO: 3:

NACRAARTTN CCRAARTC                                                      18

(2) INFORMATION FOR SEQ ID NO: 4:

    (i) SEQUENCE CHARACTERISTICS:
        (A) LENGTH: 20 base pairs
        (B) TYPE: nucleic acid
        (C) STRANDEDNESS: single

```
                    (D)  TOPOLOGY:  linear

               (ii)  MOLECULE TYPE:  DNA (genomic)

               (iii)  HYPOTHETICAL:  YES

               (iii)  ANTI-SENSE:  NO


               (ix)  FEATURE:
                    (A)  NAME/KEY:  misc_feature
                    (B)  LOCATION:  1..20
                    (D)  OTHER INFORMATION:  /label= primer

               (ix)  FEATURE:
                    (A)  NAME/KEY:  misc_feature
                    (B)  LOCATION:  3
                    (D)  OTHER INFORMATION:  N is Inosine

               (ix)  FEATURE:
                    (A)  NAME/KEY:  misc_feature
                    (B)  LOCATION:  12
                    (D)  OTHER INFORMATION:  N is Inosine


               (xi)  SEQUENCE DESCRIPTION:  SEQ ID NO: 4:

     CCNGAYCARC CNCARATHAC                                              20

     (2)  INFORMATION FOR SEQ ID NO: 5:

               (i)  SEQUENCE CHARACTERISTICS:
                    (A)  LENGTH:  58 base pairs
                    (B)  TYPE:  nucleic acid
                    (C)  STRANDEDNESS:  single
                    (D)  TOPOLOGY:  linear

               (ii)  MOLECULE TYPE:  DNA (genomic)

               (iii)  HYPOTHETICAL:  NO

               (iii)  ANTI-SENSE:  NO


               (ix)  FEATURE:
                    (A)  NAME/KEY:  misc_feature
                    (B)  LOCATION:  1..58
                    (D)  OTHER INFORMATION:  /standard_name= "probe
                         complementary to residues 228 to 232 of SEQ ID NO
                         1"


               (xi)  SEQUENCE DESCRIPTION:  SEQ ID NO: 5:

     GTCTGGAGCT GGGCCCCAGC TGCCGGAAGA GGACCAAGCC TGTGATCTGC GGCTGATC     58

     (2)  INFORMATION FOR SEQ ID NO: 6:

               (i)  SEQUENCE CHARACTERISTICS:
                    (A)  LENGTH:  20 base pairs
                    (B)  TYPE:  nucleic acid
                    (C)  STRANDEDNESS:  single
                    (D)  TOPOLOGY:  linear

               (ii)  MOLECULE TYPE:  DNA (genomic)
```

```
(iii)  HYPOTHETICAL: NO

(iii)  ANTI-SENSE: YES


(ix)  FEATURE:
      (A)  NAME/KEY: misc_feature
      (B)  LOCATION: 1..20
      (D)  OTHER INFORMATION: /standard_name= "primer
           corresponding to nucleotides 335 to 354 of seqid
           no1"


(xi)  SEQUENCE DESCRIPTION: SEQ ID NO: 6:

GGCTGTACCC TGCAGACTGC                                                    20

(2)  INFORMATION FOR SEQ ID NO: 7:

     (i)  SEQUENCE CHARACTERISTICS:
          (A)  LENGTH: 21 amino acids
          (B)  TYPE: amino acid
          (C)  STRANDEDNESS: single
          (D)  TOPOLOGY: linear

    (ii)  MOLECULE TYPE: peptide

     (v)  FRAGMENT TYPE: N-terminal



    (xi)  SEQUENCE DESCRIPTION: SEQ ID NO: 7:

Met Ser Asp Thr Gly Glu Ser Gly Val Asp Leu Ala Asp Xaa Leu Xaa
1               5                   10                  15

Leu Val Glu Lys Ile
            20

(2)  INFORMATION FOR SEQ ID NO: 8:

     (i)  SEQUENCE CHARACTERISTICS:
          (A)  LENGTH: 41 amino acids
          (B)  TYPE: amino acid
          (C)  STRANDEDNESS: single
          (D)  TOPOLOGY: linear

    (ii)  MOLECULE TYPE: peptide

     (v)  FRAGMENT TYPE: N-terminal



    (xi)  SEQUENCE DESCRIPTION: SEQ ID NO: 8:

Met Ser Asp Thr Gly Glu Ser Gly Val Asp Leu Ala Asp Arg Leu Asp
1               5                   10                  15

Leu Val Glu Lys Ile Gly Asp Thr His Ser Lys Asp Leu Glu Ile Xaa
            20                  25                  30

Met Xaa Leu Xaa Lys Gln Xaa Xaa Ala
        35                  40

(2)  INFORMATION FOR SEQ ID NO: 9:
```

```
        (i) SEQUENCE CHARACTERISTICS:
            (A) LENGTH: 7 amino acids
            (B) TYPE: amino acid
            (C) STRANDEDNESS: single
            (D) TOPOLOGY: linear

       (ii) MOLECULE TYPE: peptide

        (v) FRAGMENT TYPE: N-terminal


       (xi) SEQUENCE DESCRIPTION: SEQ ID NO: 9:

       Met Ser Asp Thr Gly Glu Ser
       1               5

(2)  INFORMATION FOR SEQ ID NO: 10:

        (i) SEQUENCE CHARACTERISTICS:
            (A) LENGTH: 20 amino acids
            (B) TYPE: amino acid
            (C) STRANDEDNESS: single
            (D) TOPOLOGY: linear

       (ii) MOLECULE TYPE: peptide

        (v) FRAGMENT TYPE: N-terminal



       (xi) SEQUENCE DESCRIPTION: SEQ ID NO: 10:

       Met Ser Asp Thr Gly Glu Ser Gly Val Asp Leu Ala Asp Arg Leu Asp
       1               5                   10                  15

       Leu Val Glu Lys
                   20

(2)  INFORMATION FOR SEQ ID NO: 11:

        (i) SEQUENCE CHARACTERISTICS:
            (A) LENGTH: 25 amino acids
            (B) TYPE: amino acid
            (C) STRANDEDNESS: single
            (D) TOPOLOGY: linear

       (ii) MOLECULE TYPE: peptide

        (v) FRAGMENT TYPE: internal



       (xi) SEQUENCE DESCRIPTION: SEQ ID NO: 11:

       Leu Pro Pro Asp Gln Pro Gln Ile Thr Gly Leu Val Leu Phe Arg Gln
       1               5                   10                  15

       Leu Gly Pro Ser Xaa Arg Leu Glu Ala
                   20                  25

(2)  INFORMATION FOR SEQ ID NO: 12:

        (i) SEQUENCE CHARACTERISTICS:
            (A) LENGTH: 44 amino acids
            (B) TYPE: amino acid
            (C) STRANDEDNESS: single
```

```
        (D) TOPOLOGY: linear

    (ii) MOLECULE TYPE: peptide

    (v) FRAGMENT TYPE: internal


    (xi) SEQUENCE DESCRIPTION: SEQ ID NO: 12:

    Leu Pro Pro Asp Gln Pro Gln Ile Thr Gly Leu Val Leu Phe Arg Gln
    1               5               10              15

    Leu Gly Pro Ser Ser Arg Leu Glu Ala Ser Phe Asn Leu Glu Gly Phe
                20              25              30

    Pro Ala Glu Gln Asn Xaa Ser Xaa Xaa Ala Ile His
            35              40

(2) INFORMATION FOR SEQ ID NO: 13:

    (i) SEQUENCE CHARACTERISTICS:
        (A) LENGTH: 25 amino acids
        (B) TYPE: amino acid
        (C) STRANDEDNESS: single
        (D) TOPOLOGY: linear

    (ii) MOLECULE TYPE: peptide

    (v) FRAGMENT TYPE: internal


    (xi) SEQUENCE DESCRIPTION: SEQ ID NO: 13:

    Gly Leu Ala Thr Ser Leu Ala Gly Pro His Ser Ile Leu Gly Arg Ala
    1               5               10              15

    Val Val Val Xaa Ala Xaa Glu Asp Asp
                20              25

(2) INFORMATION FOR SEQ ID NO: 14:

    (i) SEQUENCE CHARACTERISTICS:
        (A) LENGTH: 17 amino acids
        (B) TYPE: amino acid
        (C) STRANDEDNESS: single
        (D) TOPOLOGY: linear

    (ii) MOLECULE TYPE: peptide

    (v) FRAGMENT TYPE: internal


    (xi) SEQUENCE DESCRIPTION: SEQ ID NO: 14:

    Leu Ala Xaa Xaa Val Val Gly Thr Ser Asn Ser Glu Ala Xaa Glu Ser
    1               5               10              15

    Gln


(2) INFORMATION FOR SEQ ID NO: 15:

    (i) SEQUENCE CHARACTERISTICS:
```

```
              (A) LENGTH: 7 amino acids
              (B) TYPE: amino acid
              (C) STRANDEDNESS: single
              (D) TOPOLOGY: linear

        (ii) MOLECULE TYPE: peptide

         (v) FRAGMENT TYPE: internal



        (xi) SEQUENCE DESCRIPTION: SEQ ID NO: 15:

        Phe Gly Asn Phe Val Val Arg
        1               5

    (2) INFORMATION FOR SEQ ID NO: 16:

         (i) SEQUENCE CHARACTERISTICS:
              (A) LENGTH: 25 amino acids
              (B) TYPE: amino acid
              (C) STRANDEDNESS: single
              (D) TOPOLOGY: linear

        (ii) MOLECULE TYPE: peptide

         (v) FRAGMENT TYPE: internal



        (xi) SEQUENCE DESCRIPTION: SEQ ID NO: 16:

        Leu Xaa Gln Gly Xaa Glu Ser Thr Gly Pro Xaa Tyr Xaa Pro Leu Gly
        1               5                   10                  15

        Val Pro Xaa Pro Gln Xaa Pro Gly Asp
                    20                  25

    (2) INFORMATION FOR SEQ ID NO: 17:

         (i) SEQUENCE CHARACTERISTICS:
              (A) LENGTH: 20 amino acids
              (B) TYPE: amino acid
              (C) STRANDEDNESS: single
              (D) TOPOLOGY: linear

        (ii) MOLECULE TYPE: peptide

         (v) FRAGMENT TYPE: internal



        (xi) SEQUENCE DESCRIPTION: SEQ ID NO: 17:

        Leu Gly Xaa Gly Xaa Asn Gln Ala Ser Val Xaa Asn Gly Asn Ala Gly
        1               5                   10                  15

        Xaa Xaa Leu Ala
                    20

    (2) INFORMATION FOR SEQ ID NO: 18:

         (i) SEQUENCE CHARACTERISTICS:
              (A) LENGTH: 23 amino acids
              (B) TYPE: amino acid
              (C) STRANDEDNESS: single
```

```
        (D) TOPOLOGY: linear

    (ii) MOLECULE TYPE: peptide

     (v) FRAGMENT TYPE: C-terminal



    (xi) SEQUENCE DESCRIPTION: SEQ ID NO: 18:

    Ser Glu Ala Trp Glu Ser Gln Thr Lys Glu Arg Lys Lys Arg Arg Arg
    1                   5                  10                  15

    Glu Ser Glu Cys Lys Thr Thr
                    20

(2) INFORMATION FOR SEQ ID NO: 19:

    (i) SEQUENCE CHARACTERISTICS:
        (A) LENGTH: 4 amino acids
        (B) TYPE: amino acid
        (C) STRANDEDNESS: single
        (D) TOPOLOGY: linear

    (ii) MOLECULE TYPE: peptide

     (v) FRAGMENT TYPE: C-terminal



    (xi) SEQUENCE DESCRIPTION: SEQ ID NO: 19:

    Gly Asn Ala Gly
    1
```